# EUROPEAN PATENT SPECIFICATION

(11) **EP 1 141 306 B1**
(45) Date of publication and mention of the grant of the patent: **07.05.2008**
(21) Application number: 99960748.4
(22) Date of filing: 20.12.1999
(51) Int. Cl.: C12N 15/31, C12N 15/62, C07K 14/315, A61K 39/09

(54) **STREPTOCOCCUS ANTIGENS**
STREPTOCOCCUS ANTIGENE
ANTIGENES DE STREPTOCOCCUS

(30) Priority: 23.12.1998 US 113800 P
(43) Date of publication of application: 10.10.2001
(73) Proprietor: ID Biomedical Corporation, Laval, QC H7V 3S8 (CA)
(72) Inventor: HAMEL, Josée, Sillery, Québec G1T 1N6 (CA); BRODEUR, Bernard, R., Sillery, Québec G1T 1N6 (CA); PINEAU, Isabelle, Sainte-Foy, Québec G1V 3Y7 (CA); MARTIN, Denis, St-Augustin-DesMaures, Québec G3A 1E9 (CA); RIOUX, Clément, Ile Bizard, Québec H9E 1J1 (CA); CHARLAND, Nathalie, Charny, Québec J6X 3N6 (CA)
(74) Representative: Johnston, Caroline Louise
(86) International application number: PCT/CA1999/001218
(87) International publication number: WO 2000/039299

(56) References cited:
- WO-A-00/06737
- WO-A-00/06738
- WO-A-00/17370
- WO-A-98/18930
- WO-A-98/18931
- WO-A-99/15675

## Description

### FIELD OF THE INVENTION

The present invention is related to antigens, more particularly protein antigens of streptococcus pneumoniaepathogen which are useful as vaccine components for therapy and/or prophylaxis.

### BACKGROUND OF THE INVENTION

S. pneumoniae is an important agent of disease in man especially among infants, the elderly and immunocompromised persons. It is a bacterium frequently isolated from patients with invasive diseases such as bacteraemia/septicaemia, pneumonia, meningitis with high morbidity and mortality throughout the world. Even with appropriate antibiotic therapy, pneumococcal infections still result in many deaths. Although the advent of antimicrobial drugs has reduced the overall mortality from pneumococcal disease, the presence of resistant pneumococcal organisms has become a major problem in the world today. Effective pneumococcal vaccines could have a major impact on the morbidity and mortality associated with S. pneumoniae disease. Such vaccines would also potentially be useful to prevent otitis media in infants and young children.

Efforts to develop a pneumococcal vaccine have generally concentrated on generating immune responses to the pneumococcal capsular polysaccharide. More than 80 pneumococcal capsular serotypes have been identified on the basis of antigenic differences. The currently available pneumococcal vaccine, comprising 23 capsular polysaccharides that most frequently caused disease, has significant shortcomings related primarily to the poor immunogenicity of some capsular polysaccharides, the diversity of the serotypes and the differences in the distribution of serotypes over time, geographic areas and age groups. In particular, the failure of existing vaccines and capsular conjugate vaccines currently in development to protect young children against all serotypes spurres evaluation of other S. pneumoniae components. Although immunogenicity of capsular polysaccharides can be improved, serotype specificity will still represent a major limitation of polysaccharide-based vaccines. The use of a antigenically conserved immunogenic pneumococcal protein antigen, either by itself or in combination with additional components, offers the possibility of a protein-based pneumococcal vaccine.

PCT Publication number WO98/18930 published may 7 1998 entitled *"Streptococcus Pneumoniae* antigens and vaccines" describes certain polypeptides which are claimed to be antigenic. However, no biological activity of these polypeptides is reported. WO 98/18931, published on the same day is entitled "Streptococcus pneumoniae polynucleotides and sequences".

Therefore their remains an unmet need for Streptococcus antigens that may be used as vaccine components for the prophylaxis and/or therapy of Streptococcus infection.

### SUMMARY OF THE INVENTION

According to one aspect, the present invention provides an isolated polynucleotide encoding a polypeptide having at least 95% identity to a second polypeptide comprising a sequence chosen from **SEQ ID NOs :2, 8, 10, 16, 55,** 64-66 or 78 wherein the polypeptide elicits an anti-streptococcal immune response when administered to an individual.

In other aspects, there are provided vectors comprising polynucleotides of the invention operably linked to an expression control region, as well as host cells transfected with said vectors and methods of producing polypeptides comprising culturing said host cells under conditions suitable for expression.

Said host cells are not human embryonic stem cells or human germ cells.

In yet another aspect, there are provided novel polypeptides encoded by polynucleotides of the invention.

### BRIEF DESCRIPTION OF THE DRAWINGS

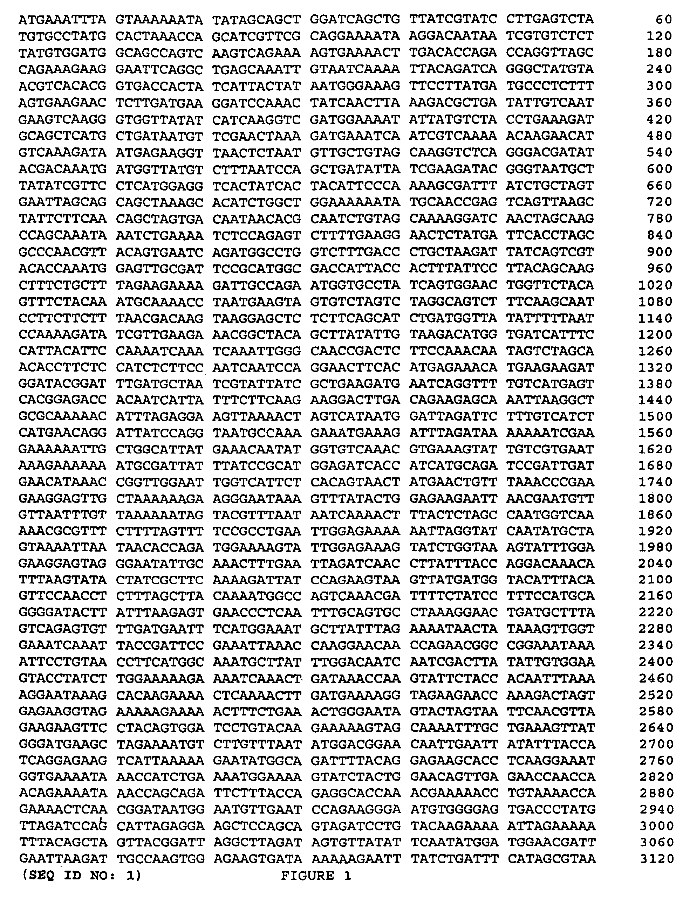

Figure 1 is the DNA sequence of BVH-3 gene; **SEQ ID NO: 1**.

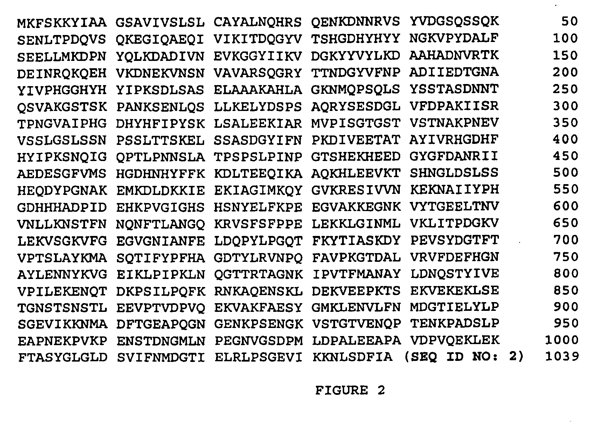

Figure 2 is the amino acid sequence of BVH-3 protein; **SEQ ID NO: 2.**

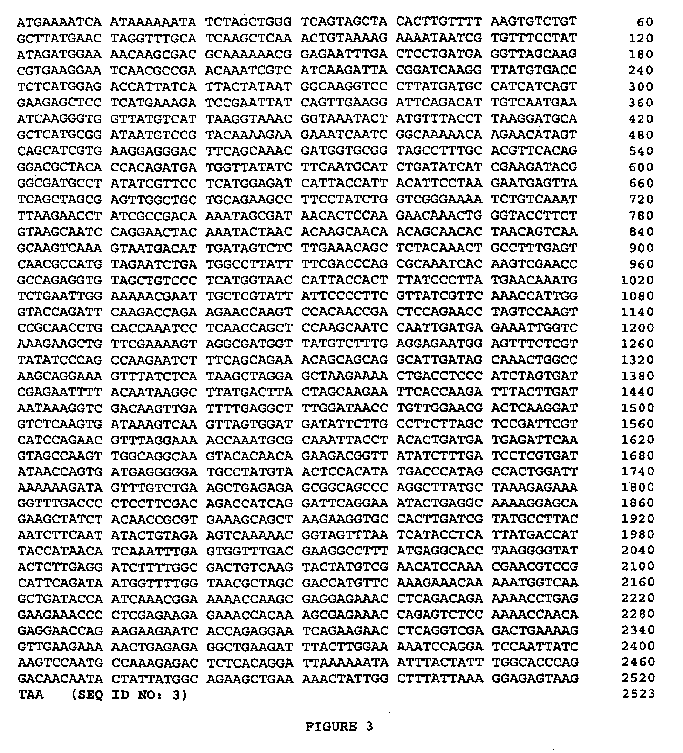

Figure 3 is the DNA sequence of BVH-11 gene; **SEQ ID NO: 3.**

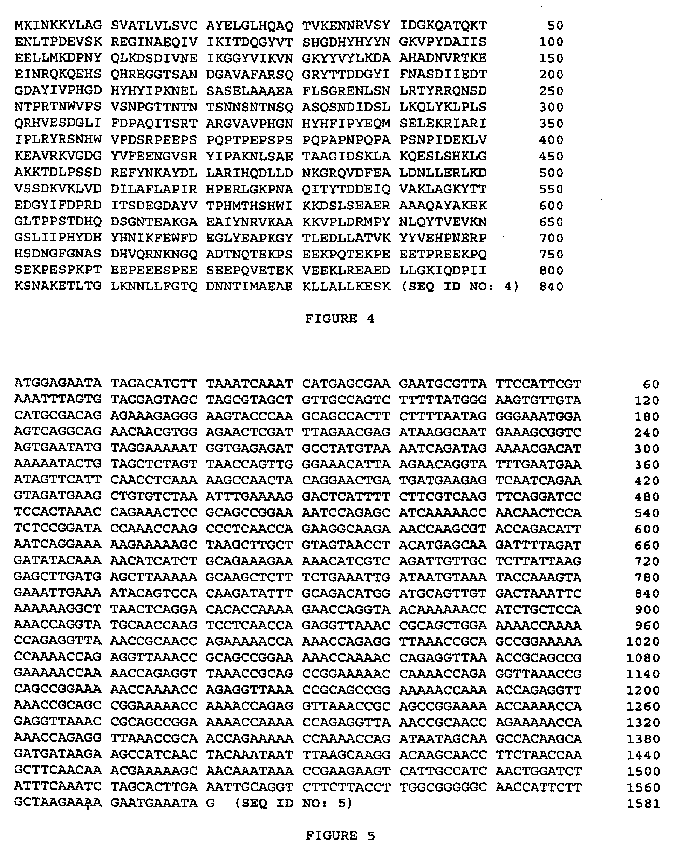

Figure 4 is the amino acid sequence of BVH-11 protein; **SEQ ID NO: 4.**
Figure 5 is the DNA sequence of BVH-28 gene; **SEQ ID NO: 5.**

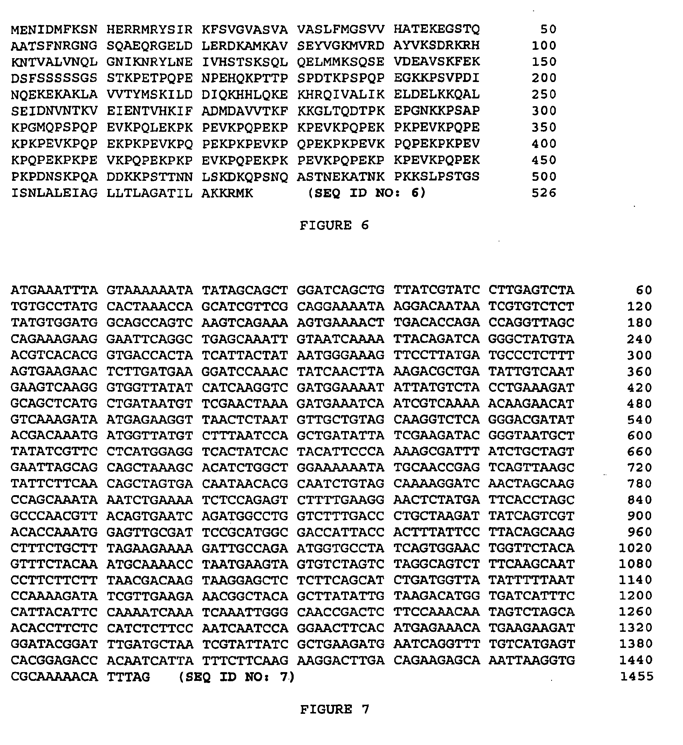

Figure 6 is the amino acid sequence of BVH-28 protein; **SEQ ID NO: 6**.
Figure 7 is the DNA sequence of BVH-3A gene which corresponds to the 5' terminal end of BVH-3; **SEQ ID NO:** 7.

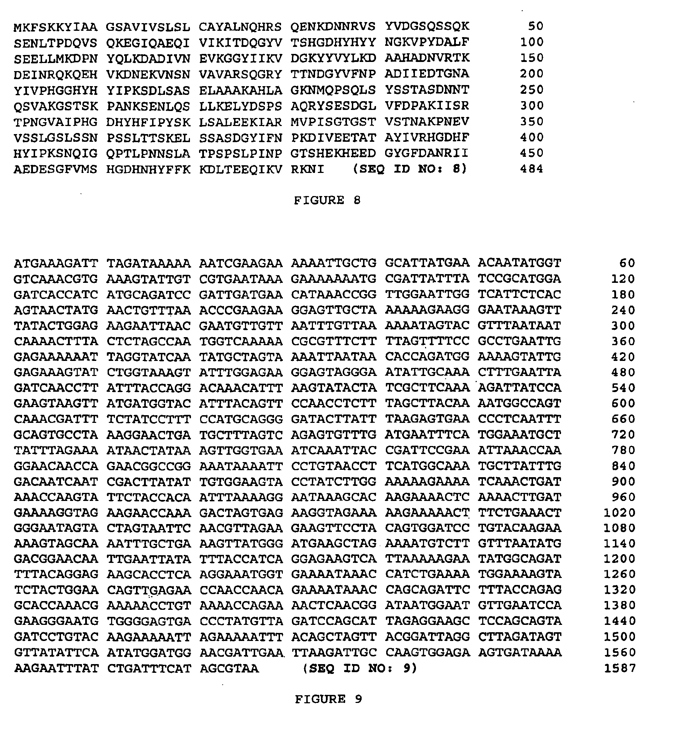

Figure 8 is the amino acid sequence of BVH-3A protein; **SEQ ID NO: 8.**
Figure 9 is the DNA sequence of BVH-3B gene which corresponds to the 3' terminal end of BVH-3; **SEQ ID NO : 9.**

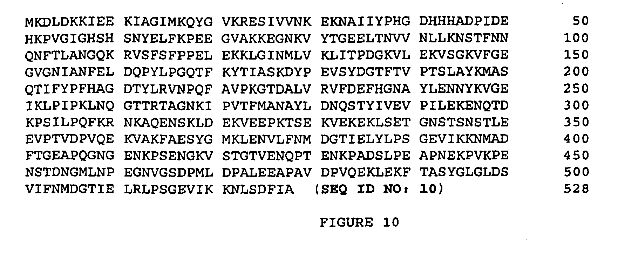

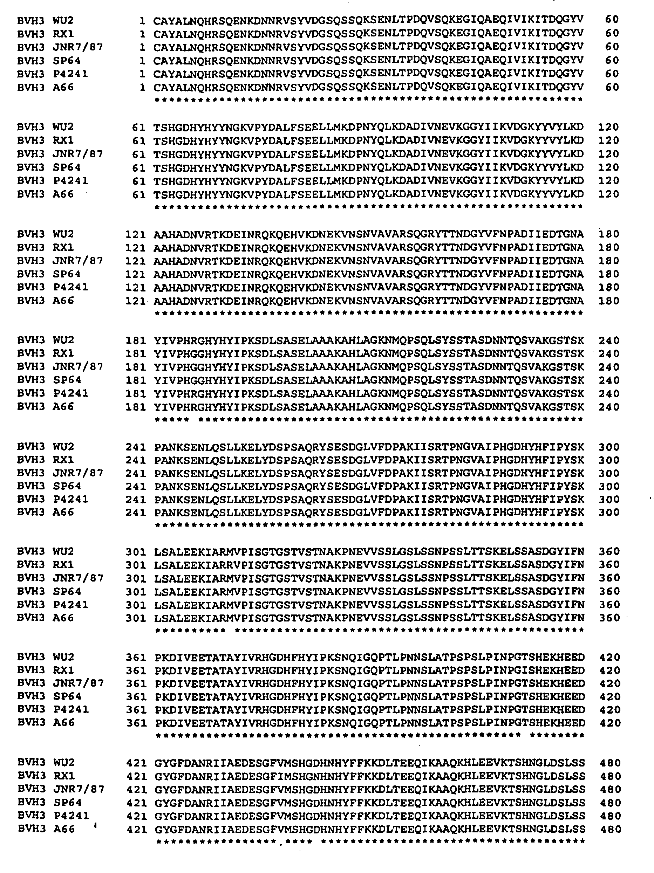

Figure 10 is the amino acid sequence of BVH-3B protein; **SEQ ID NO: 10.**
Figure 11 depicts the comparison of the predicted amino acid sequences of the BVH-3 open reading frames from WU2, RX1, JNR.7/87, SP64, P4241 and A66 S. pneumoniae strains by using the program Clustal W from MacVector sequence analysis software (version 6.5). Underneath the alignment, there is a consensus line where * and . characters indicate identical and similar amino acid residues, respectively.
Figure 12 depicts the comparison of the predicted amino acid sequences of the BVH-11 open reading frames from WU2, Rx1, JNR.7/87, SP64, P4241, A66 and SP63 S. pneumoniae strains by using the program Clustal W from MacVector sequence analysis software (version 6.5). Underneath the alignment, there is a consensus line where * and characters indicate identical and similar amino acid residues, respectively.
Figure 13 depicts the comparison of the predicted amino acid sequences of the BVH-11 proteins from various S. pneumoniae strains. The degrees of identity (I) and similarity (S) were determined by using the program Clustal W from MacVector sequence analysis software (version 6.5).

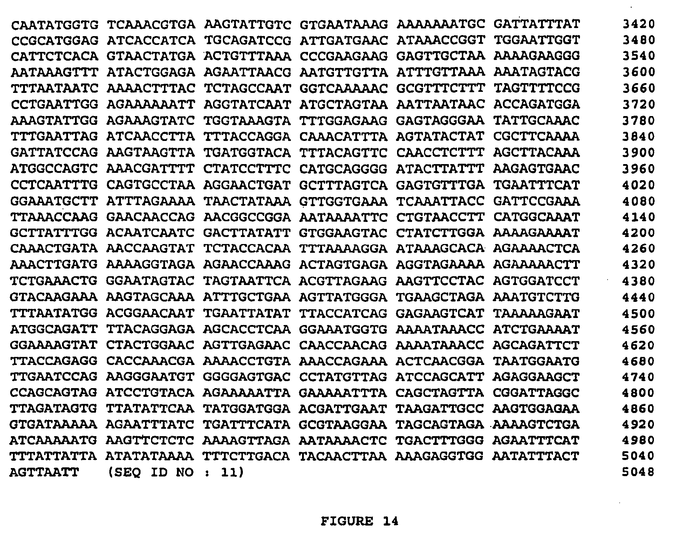

Figure 14 is a DNA sequence containing the complete BVH-3 gene (open reading frame "ORF" at nucleotides 1777 to 4896); **SEQ ID NO: 11**.

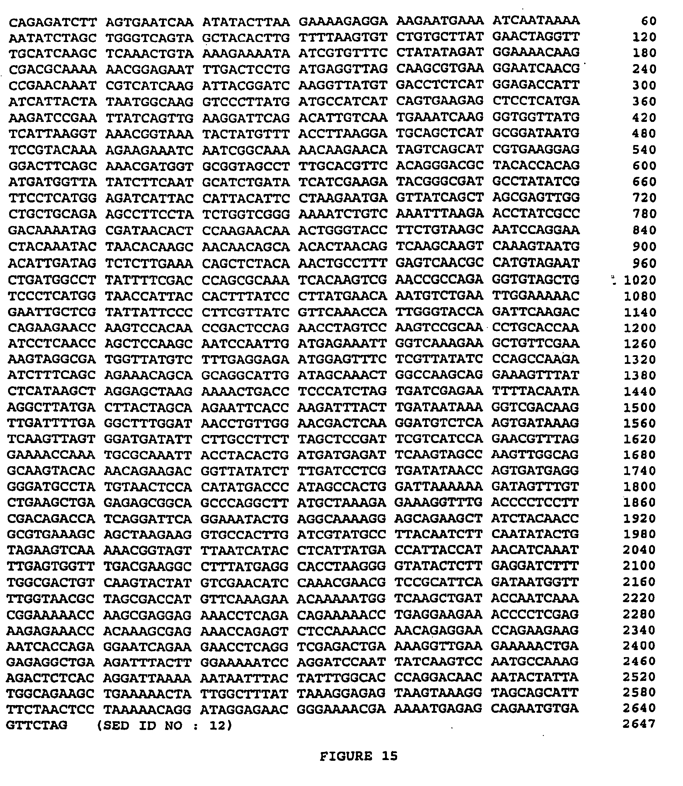

Figure 15 is a DNA sequence containing the complete BVH-11 gene (ORF at nucleotides 45 to 2567); **SEQ ID NO: 12.**

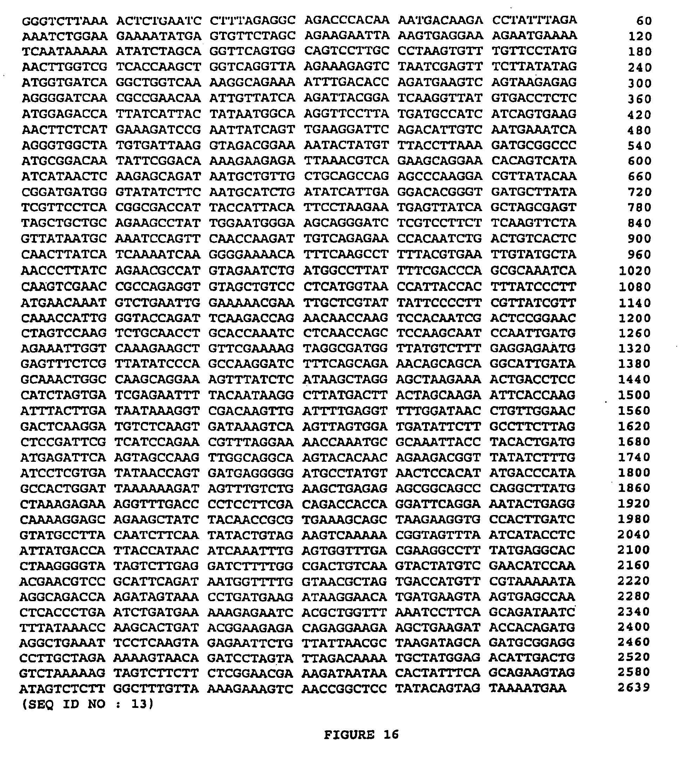

Figure 16 is a DNA sequence containing the complete BVH-11-2 gene (ORF at nucleotides 114 to 2630); **SEQ ID NO: 13.**

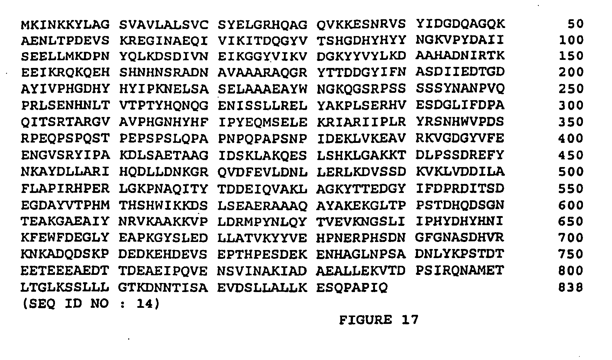

Figure 17 is the amino acid sequence of BVH-11-2 protein; **SEQ ID NO: 14.**

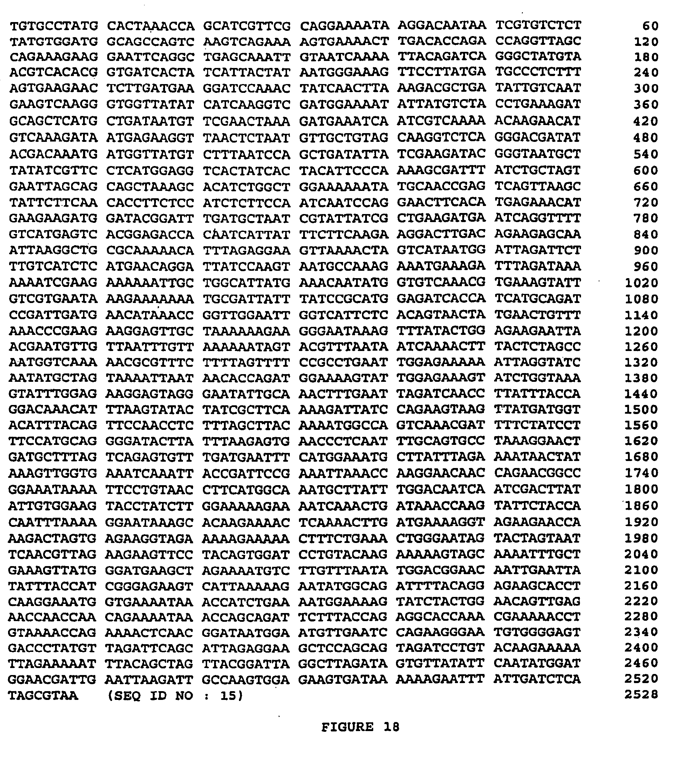

Figure 18 is the DNA sequence of SP63 BVH-3 gene; **SEQ ID NO:15.**

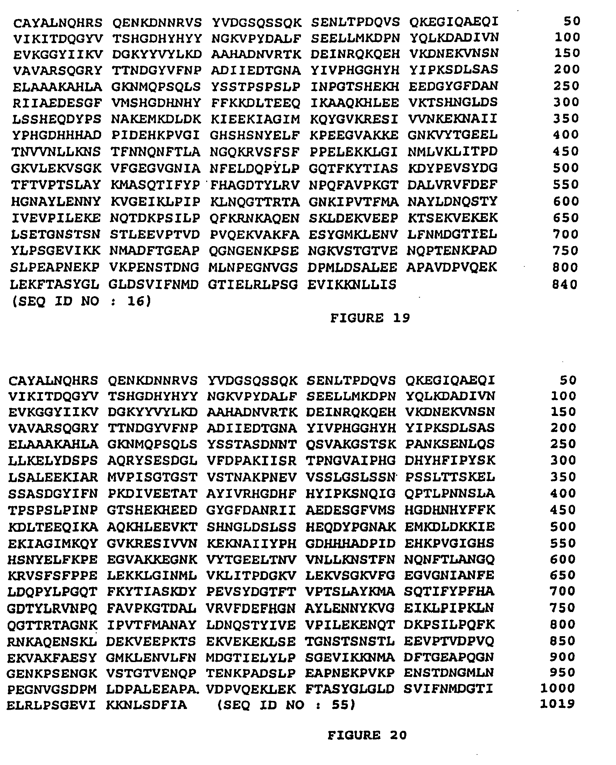

Figure 19 is the amino acid sequence of SP63 BVH-3 protein; **SEQ ID NO: 16.**
Figure 20 is the amino acid sequence of BVH-3M protein; **SEQ ID NO: 55.**

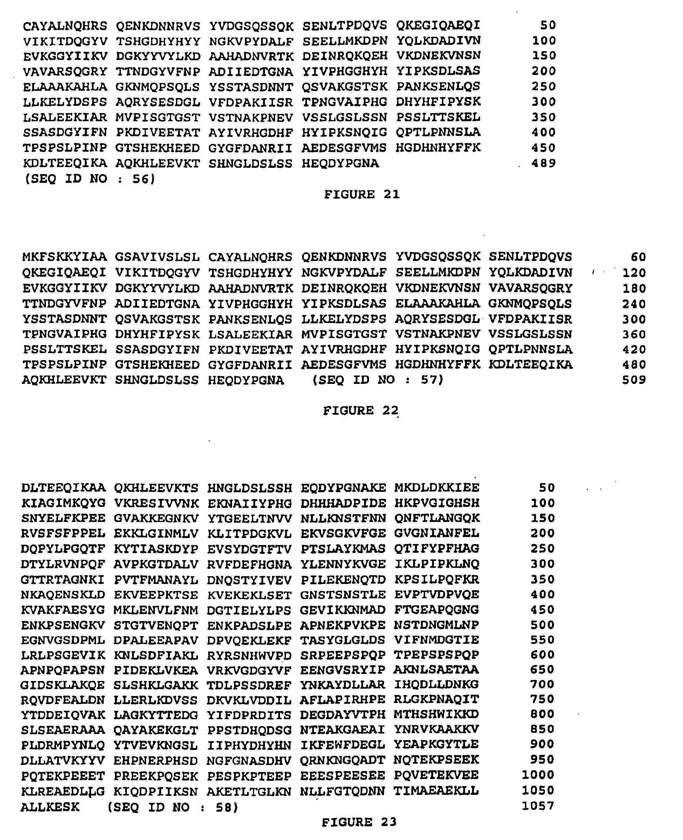

Figure 21 is the amino acid sequence of BVH-3AD protein; **SEQ ID NO: 56**.
Figure 22 is the amino acid sequence of L-BVH-3-AD protein; **SEQ ID NO: 57.**
Figure 23 is the amino acid sequence of NEW12 protein; **SEQ ID NO: 58.**

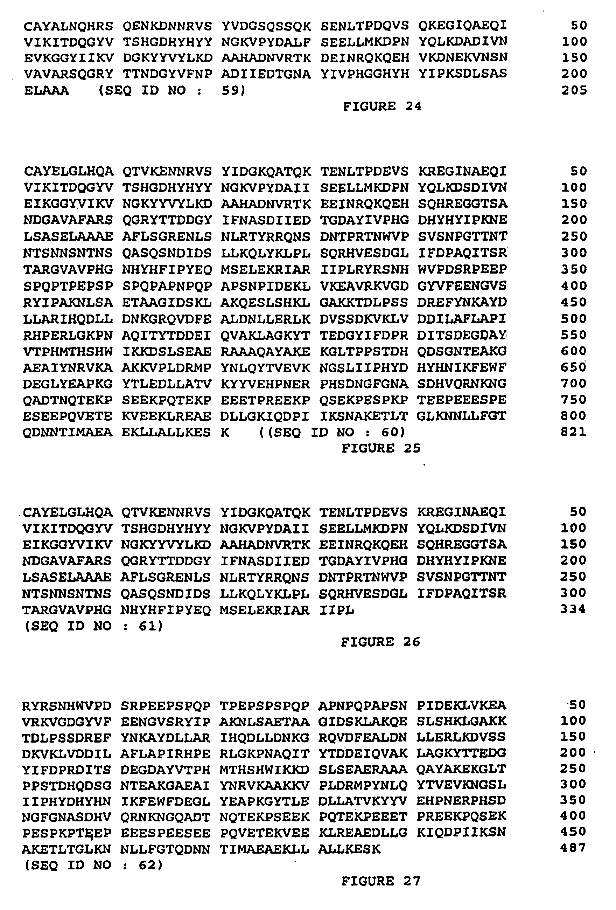

Figure 24 is the amino acid sequence of BVH-3C protein; **SEQ ID NO: 59.**
Figure 25 is the amino acid sequence of BVH-11M protein; **SEQ ID NO: 60.**
Figure 26 is the amino acid sequence of BVH-11A protein; **SEQ ID NO: 61.**
Figure 27 is the amino acid sequence of BVH-11B (also called New13) protein; **SEQ ID NO: 62.**

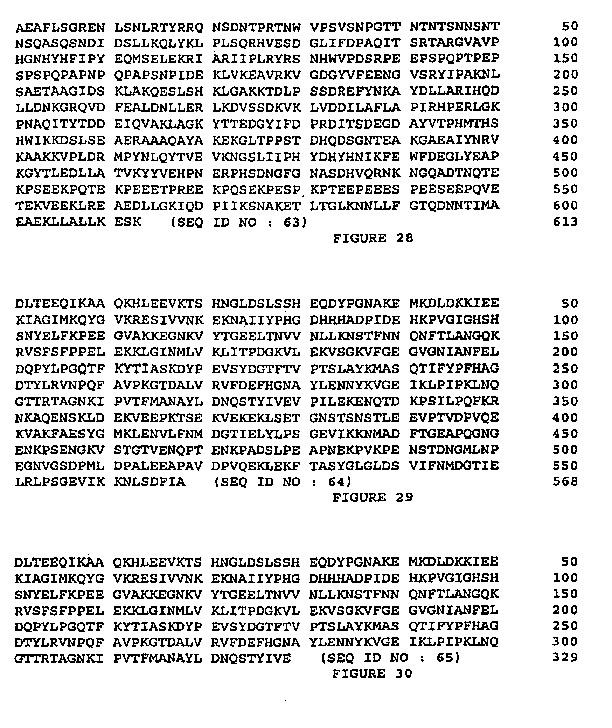

Figure 28 is the amino acid sequence of BVH-11C protein; **SEQ ID NO: 63.**
Figure 29 is the amino acid sequence of NEW1 protein; **SEQ ID NO: 64**.
Figure 30 is the amino acid sequence of NEW2 protein; **SEQ ID NO: 65.**

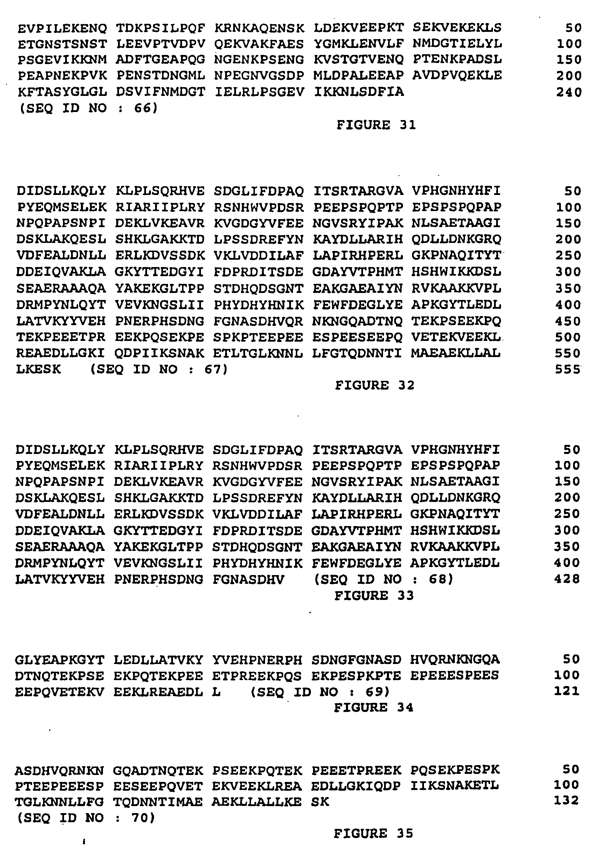

Figure 31 is the amino acid sequence of NEW3 protein; **SEQ ID NO: 66**.
Figure 32 is the amino acid sequence of NEW4 protein; **SEQ ID NO: 67**.
Figure 33 is the amino acid sequence of NEW5 protein; **SEQ ID NO: 68.**
Figure 34 is the amino acid sequence of NEW6 protein; **SEQ ID NO: 69.**
Figure 35 is the amino acid sequence of NEW7 protein; SEQ **ID NO: 70.**

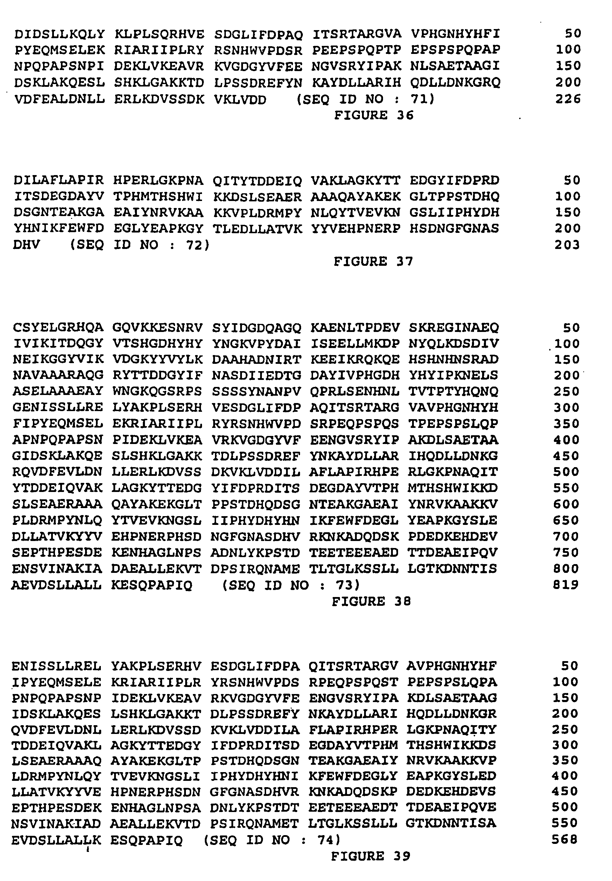

Figure 36 is the amino acid sequence of NEW8 protein; **SEQ ID NO: 71.**
Figure 37 is the amino acid sequence of NEW9 protein; **SEQ ID NO: 72.**
Figure 38 is the amino acid sequence of BVH-11-2M protein; **SEQ ID NO: 73.**
Figure 39 is the amino acid sequence of NEW10 protein; **SEQ ID NO: 74.**

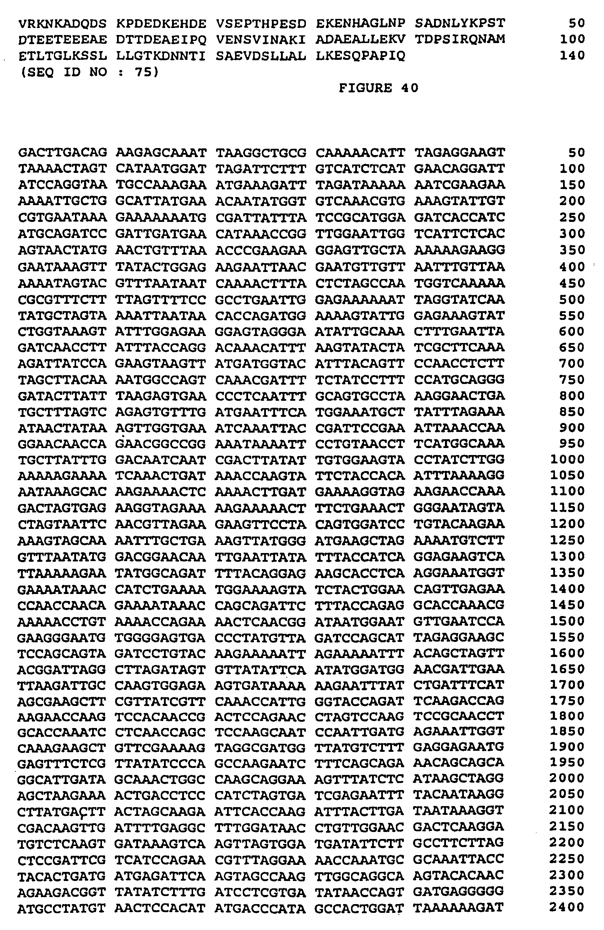

Figure 40 is the amino acid sequence of NEW11 protein; **SEQ ID NO: 75.**

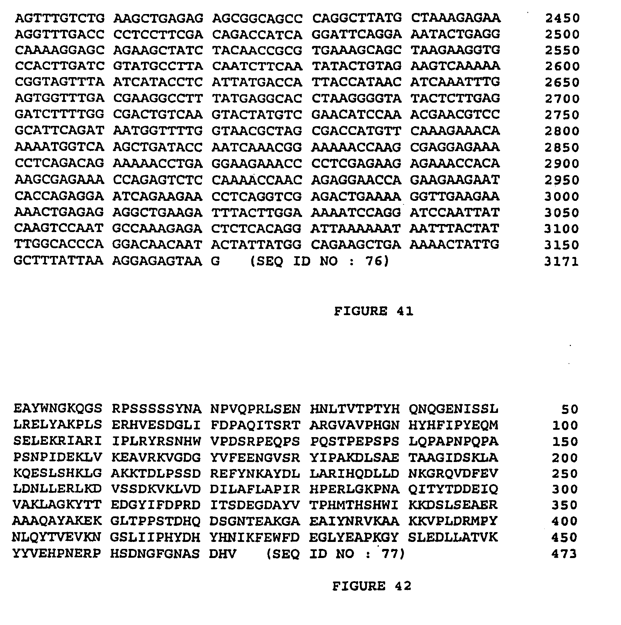

Figure 41 is the DNA sequence of NEW12 gene; **SEQ ID NO: 76.**
Figure 42 is the amino acid sequence of NEW14 protein; **SEQ ID NO: 77.**

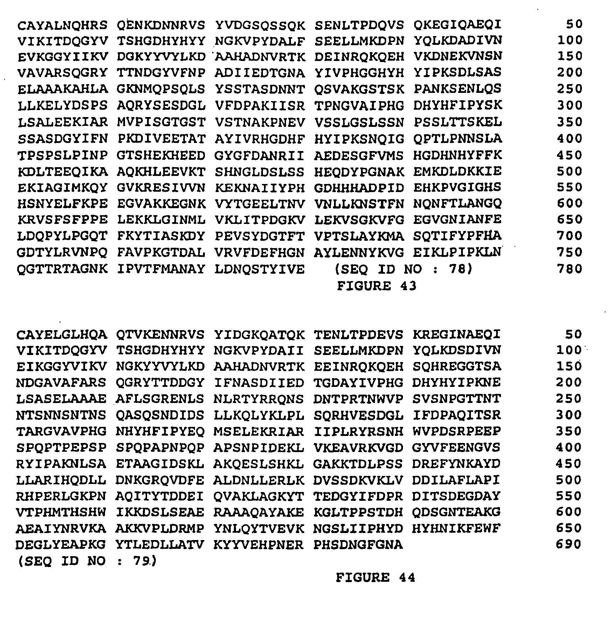

Figure 43 is the amino acid sequence of NEW15 protein; **SEQ ID NO: 78.**
Figure 44 is the amino acid sequence of NEW16 protein; **SEQ ID NO: 79.**

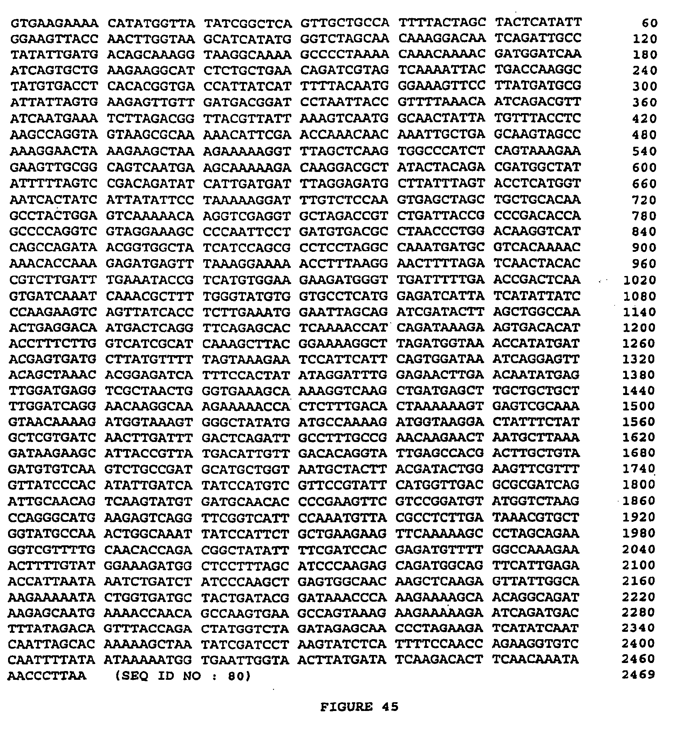

Figure 45 is the DNA sequence of GBS BVH-71 gene; **SEQ ID NO: 80.**

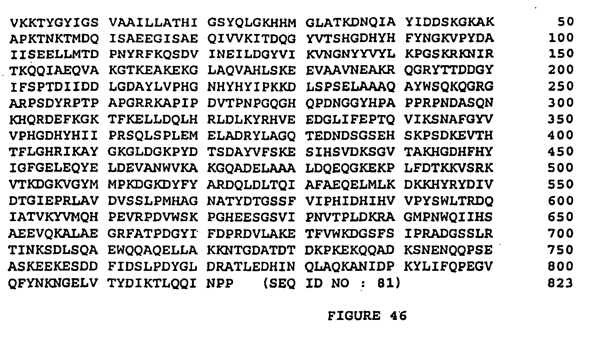

Figure 46 is the amino acid sequence of GBS BVH-71 protein; **SEQ ID NO: 81.**

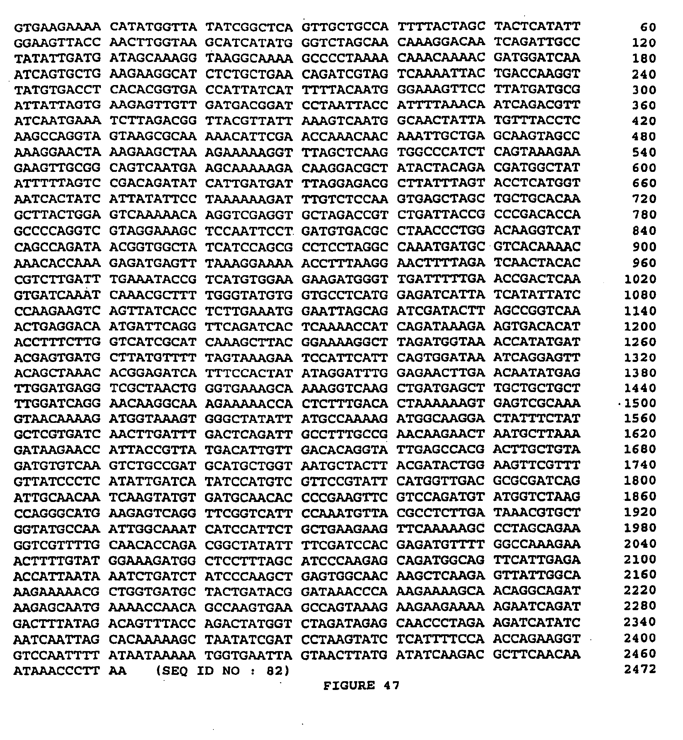

Figure 47 is the DNA sequence of GAS BVH-71 gene; **SEQ ID NO:82.**

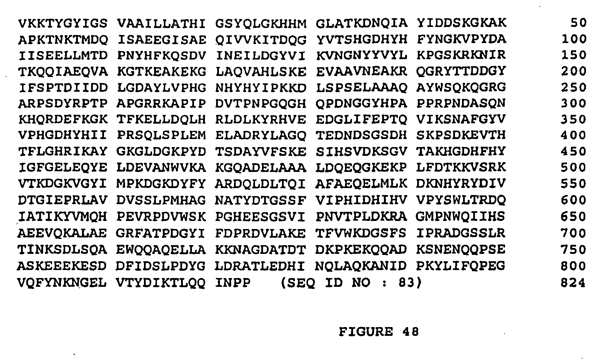

Figure 48 is the amino acid sequence of GAS BVH-71 protein; **SEQ ID NO:83.**

### DETAILED DESCRIPTION OF THE INVENTION

According to one aspect, the present invention provides an isolated polynucleotide encoding a polypeptide having at least 95% identity to any one of **SEQ ID NOs: 2, 8, 10, 16, 55,** 64-66 or 78 wherein the polypeptide elicits an anti-streptococcal immune response when administered to an individual.

According to one aspect, the present invention relates to claim 9.

In a further embodiment, the present invention also relates to chimeric polypeptides which comprise one or more polypeptides or fragments, analogs or derivatives thereof as described in the present application.

In a further embodiment, the present invention also relates to chimeric polypeptides which comprise one or more polypeptides or fragments, analogs or derivatives thereof as defined in the figures of the present application.

In a further embodiment, the present application also relates to chimeric polypeptides which comprise two or more polypeptides chosen from **SEQ ID NOs: 2, 8, 10, 16, 55, 64-66 or 78;** provided that the polypeptides or fragments, analogs or derivatives thereof are linked as to form a chimeric polypeptide.

In a further embodiment, the chimeric polypeptide will comprise between 2 and 5 polypeptides.

In a further embodiment, the chimeric polypeptide will comprise between 2 and 4 polypeptides.

In a further embodiment, the chimeric polypeptide will comprise between 2 and 3 polypeptides.

In a further embodiment, the chimeric polypeptide will comprise 2 polypeptides.

In a further embodiment, there is provided a chimeric polypeptide of formula (I) which elicits an anti-streptococcal immune response when administered to an individual: **A**-(**B**)ₘ-(**C**)ₙ-**D** (I)

Wherein;
**m** is 0 or 1,
**n** is 0 or 1,
**A** is chosen from **SEQ ID NOs: 2, 8, 10, 16, 55, 64-66 or 78;**
**B** is chosen from **SEQ ID NOs: 2**, **8, 10, 16, 55, 64-66 or 78;**
**C** is chosen from **.SEQ ID NOs: 2**, **8**, **10**, **16**, **55, 64-66 or 78;** and
**D** is chosen from **SEQ ID NOs: 2, 8**, **10**, **16**, **55, 64-66 or 78;**

In one embodiment, chimeric polypeptides of the present invention comprise those wherein the following embodiments are present, either independently or in combination.

In a further embodiment, **A** is **SEQ ID NOs :10**, **64,** or fragments, analogs or derivatives thereof.

In a further embodiment, **A** is **SEQ ID NO :10** or fragments, analogs or derivatives thereof.

In a further embodiment, **A** is **SEQ ID NO :64** or fragments, analogs or derivatives thereof.

In a further embodiment, **B** is **SEQ ID NO :10** or fragments, analogs or derivatives thereof.

In a further embodiment, **C** is **SEQ ID NOs :10, 64.**

In a further embodiment, **C** is **SEQ ID NO :10** or fragments, analogs or derivatives thereof.

In a further embodiment, **C** is **SEQ ID NO : 62** or fragments, analogs or derivatives thereof.

In a further embodiment, **D** is **SEQ ID NO :10, 58, 62, 64, 67, 68, 74, 77** or fragments, analogs or derivatives thereof.

In a further embodiment, **D** is **SEQ ID NO :10** or fragments, analogs or derivatives thereof.

In a further embodiment, **D** is **SEQ ID NO :64** or fragments, analogs or derivatives thereof.

In a further embodiment, **m** is 0.
In a further embodiment, **n** is 0.

In a further embodiment, **m** and **n** are 0.

In a further embodiment, **m** and **n** are 0, **A** is **SEQ ID NO:64** or fragments, analogs or derivatives thereof, **B** is **SEQ ID NO:62** or fragments, analogs or derivatives thereof.
In a further embodiment, m and n are 0, **A** is **SEQ ID NO:62** or fragments, analogs or derivatives thereof, **B** is **SEQ ID NO:64** or fragments, analogs or derivatives thereof.

In accordance with the present invention, all nucleotides encoding polypeptides and chimeric polypeptides are within the scope of the present invention.

In a further embodiment, the polypeptides or chimeric polypeptides in accordance with the present invention are antigenic.

In a further embodiment, the polypeptides or chimeric polypeptides in accordance with the present invention can elicit an immune response in an individual.

In a further embodiment, the present invention also relates to polypeptides which are able to raise antibodies having binding specificity to the polypeptides or chimeric polypeptides of the present invention as defined above.

An antibody that " has binding specificity" is an antibody that recognizes and binds the selected polypeptide but which does not substantially recognize and bind other molecules in a sample, e.g., a biological sample, which naturally includes the selected peptide. Specific binding can be measured using an ELISA assay in which the selected polypeptide is used as an antigen.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety. In case of conflict, the present specification, including definitions, will control. In addition, the materials, methods, and examples are illustrative only and not intended to be limiting.

As used herein, "fragments", "derivatives" or "analogs" of the polypeptides of the invention include those polypeptides in which one or more of the amino acid residues are substituted with a conserved or non-conserved amino acid residue (preferably conserved) and which may be natural or unnatural. In one embodiment, derivatives and analogs of polypeptides of the invention will have about 70% identity with those sequences illustrated in the figures or fragments thereof. That is, 70% of the residues are the same. In a further embodiment, polypeptides will have greater than 75% homology. In a further embodiment, polypeptides will have greater than 80% homology. In a further embodiment, polypeptides will have greater than 85% homology. In a further embodiment, polypeptides will have greater than 90% homology. In a further embodiment, polypeptides will have greater than 95% homology. In a further embodiment, polypeptides will have greater than 99% homology. In a further embodiment, derivatives and analogs of polypeptides of the invention will have fewer than about 20 amino acid residue substitutions, modifications or deletions and more preferably less than 10. Preferred substitutions are those known in the art as conserved i.e. the substituted residues share physical or chemical properties such as hydrophobicity, size, charge or functional groups.

In accordance with the present invention, polypeptides of the invention include both polypeptides and chimeric polypeptides.

Also included are polypeptides which have fused thereto other compounds which alter the polypeptides biological or pharmacological properties i.e. polyethylene glycol (PEG) to increase half-life; leader or secretory amino acid sequences for ease of purification; prepro- and pro-sequences; and (poly)saccharides.

Furthermore, in those situations where amino acid regions are found to be polymorphic, it may be desirable to vary one or more particular amino acids to more effectively mimic the different epitopes of the different streptococcus strains.

Moreover, the polypeptides of the present invention can be modified by terminal -NH₂ acylation (eg. by acetylation, or thioglycolic acid amidation, terminal carbosy amidation, e.g. with ammonia or methylamine) to provide stability, increased hydrophobicity for linking or binding to a support or other molecule.

Also contemplated are hetero and homo polypeptide multimers of the polypeptide fragments, analogues and derivatives.
These polymeric forms include, for example, one or more polypeptides that have been cross-linked with cross-linkers such as avidin/biotin, gluteraldehyde or dimethyl-superimidate. Such polymeric forms also include polypeptides containing two or more tandem or inverted contiguous sequences, produced from multicistronic mRNAs generated by recombinant DNA technology.
Preferably, a fragment, analog or derivative of a polypeptide of the invention will comprise at least one antigenic region i.e. at least one epitope.

In order to achieve the formation of antigenic polymers (i.e. synthetic multimers), polypeptides may be utilized having bishaloacetyl groups, nitroarylhalides, or the like, where the reagents being specific for thio groups.

Therefore, the link between two mercapto groups of the different peptides may be a single bond or may be composed of a linking group of at least two, typically at least four, and not more than 16, but usually not more than about 14 carbon atoms.

In a particular embodiment, polypeptide fragments, analogs and derivatives of the invention do not contain a methionine (Met) starting residue. Preferably, polypeptides will not incorporate a leader or secretory sequence (signal sequence). The signal portion of a polypeptide of the invention may be determined according to established molecular biological techniques. In general, the polypeptide of interest may be isolated from a streptococcus culture and subsequently sequenced to determine the initial residue of the mature protein and therefore the sequence of the mature polypeptide.

According to another aspect, there are provided vaccine compositions comprising one or more streptococcus polypeptides of the invention in admixture with a pharmaceutically acceptable carrier diluent or adjuvant. Suitable adjuvants include oils i.e. Freund's complete or incomplete adjuvant; salts i.e. AlK(SO₄)₂, AlNa(SO₄)₂, AlNH₄(SO₄)₂, silica, kaolin, carbon polynucleotides i.e. poly IC and poly AU. Preferred adjuvants include QuilA and Alhydrogel. Vaccines of the invention may be administered parenterally by injection, rapid infusion, nasopharyngeal absorption, dermoabsorption, or bucal or oral.
Pharmaceutically acceptable carriers also include tetanus toxoid.

Vaccine compositions of the invention are used for the treatment or prophylaxis of streptococcus infection and/or diseases and symptoms mediated by streptococcus infection as described in P.R. Murray (Ed, in chief),E.J. Baron, M.A. Pfaller, F.C. Tenover and R.H. Yolken. Manual of Clinical Microbiology, ASM Press, Washington, D.C. sixth edition, 1995, 1482p which are herein incorporated by reference. In one embodiment, vaccine compositions of the present invention are used for the treatment or prophylaxis of meningitis, otitis media, bacteremia or pneumonia. In one embodiment, vaccine compositions of the invention are used for the treatment or prophylaxis of streptococcus infection and/or diseases and symptoms mediated by *streptococcus* infection, in particular S.pneumoniae, group A *streptococcus (pyogenes),* group B *streptococcus* (GBS or *agalactiae), dysgalactiae, uberis, nocardia* as well as *Staphylococcus* aureus. In a further embodiment, the streptococcus infection is S.pneumoniae.

In a particular embodiment, vaccines are administered to those individuals at risk of streptococcus infection such as infants, elderly and immunocompromised individuals.

As used in the present application, the term " individuals" include mammals. In a further embodiment, the mammal is human.

Vaccine compositions are preferably in unit dosage form of about 0.001 to 100 ug/kg (antigen/body weight) and more preferably 0.01 to 10 µg/kg and most preferably 0.1 to 1 µg/kg 1 to 3 times with an interval of about 1 to 6 week intervals between immunizations.

According to another aspect, there are provided polynucleotides encoding polypeptides characterized by the amino'acid sequence chosen from **SEQ ID NOs: 2, 8, 10, 16, 55, 64-66 or 78**.

In one embodiment, polynucleotides are those illustrated in **SEQ ID Nos: 1, 7, 9, 15**, which may include the open reading frames (ORF), encoding polypeptides of the invention. It will be appreciated that the polynucleotide sequences illustrated in the figures may be altered with degenerate codons yet still encode the polypeptides of the invention. Accordingly the present invention further provides polynucleotides which hybridize to the polynucleotide sequences herein above described (or the complement sequences thereof) having 50% identity between sequences. In one embodiment, at least 70% identity between sequences. In one embodiment, at least 75% identity between sequences. In one embodiment, at least 80% identity between sequences. In one embodiment, at least 85% identity between sequences. In one embodiment, at least 90% identity between sequences. In a further embodiment, polynucleotides are hybridizable under stringent conditions i.e. having at least 95% identity. In a further embodiment, more than 97% identity.

In a further embodiment, polynucleotides are those illustrated in **SEQ ID NOs: 1, 7, 9, 15,** encoding polypeptides of the invention.

In a further embodiment, polynucleotides are those illustrated in **SEQ ID NOs : 1, 9, 15,** which may include the open reading frames (ORF), encoding polypeptides of the invention.

In a further embodiment, polynucleotides are those illustrated in **SEQ ID NOs : 1, 9, 15,** which may include the open reading frames (ORF), encoding polypeptides of the invention.

In a further embodiment, polynucleotides are those illustrated in **SEQ ID NOs : 1, 7, 9, 15,** which may include the open reading frames (ORF), encoding polypeptides of the invention.

In a further embodiment, polynucleotides are those illustrated in **SEQ ID NOs : 1, 7, 9, 15,** which may include the open reading frames (ORF), encoding polypeptides of the invention.

In a.further embodiment, polynucleotides are those illustrated in **SEQ ID NOs : 1, 9, 15,** which may include the open reading frames (ORF), encoding polypeptides of the invention.

In a further embodiment, polynucleotides are those illustrated in **SEQ ID NOs** : **1, 7, 9**, which may include the open reading frames (ORF), encoding polypeptides of the invention.

In a further embodiment, polynucleotides are those illustrated in **SEQ ID NO : 1,** encoding polypeptides of the invention.

In a further embodiment, polynucleotides are those illustrated in **SEQ ID NO** :**7**, encoding polypeptides of the invention.

In a further embodiment, polynucleotides are those illustrated in **SEQ ID NO :9**, encoding polypeptides of the invention.

In a further embodiment, polynucleotides are those illustrated in **SEQ ID NO :15,** encoding polypeptides of the invention.

As will be readily appreciated by one skilled in the art, polynucleotides include both DNA and RNA.

The present invention also includes polynucleotides complementary to the polynucleotides described in the present application.

In a'further aspect, polynucleotides encoding polypeptides of the invention, or fragments, analogs or derivatives thereof, may be used in a DNA immunization method. That is, they can be incorporated into a vector which is replicable and expressible upon injection thereby producing the antigenic polypeptide in vivo. For example polynucleotides may be incorporated into a plasmid vector under the control of the CMV promoter which is functional in eukaryotic cells. Preferably the vector is injected intramuscularly.

According to another aspect, there is provided a process for producing polypeptides of the invention by recombinant techniques by expressing a polynucleotide encoding said polypeptide in a host cell and recovering the expressed polypeptide product. Alternatively, the polypeptides can be produced according to established synthetic chemical techniques i.e. solution phase or solid phase synthesis of oligopeptides which are ligated to produce the full polypeptide (block ligation).

General methods for obtention and evaluation of polynucleotides and polypeptides are described in the following references: Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York; PCR Cloning Protocols, from Molecular Cloning to Genetic Engineering, Edited by White B.A., Humana Press, Totowa, New Jersey, 1997, 490 pages; Protein Purification, Principles and Practices, Scopes R.K., Springer-Verlag, New York, 3rd Edition, 1993, 380 pages; Current Protocols in Immunology, Edited by Coligan J.E. et al., John Wiley & Sons Inc., New York which are herein incorporated by reference.

For recombinant production, host cells are transfected with vectors which encode the polypeptide, and then cultured in a nutrient media modified as appropriate for activating promoters, selecting transformants or amplifying the genes.

Suitable vectors are those that are viable and replicable in the chosen host and include chromosomal, non-chromosomal and synthetic DNA sequences e.g. bacterial plasmids, phage DNA, baculovirus, yeast plasmids, vectors derived from combinations of plasmids and phage DNA. The polypeptide sequence may be incorporated in the vector at the appropriate site using restriction enzymes such that it is operably linked to an expression control region comprising a promoter, ribosome binding site (consensus region or Shine-Dalgarno sequence), and optionally an operator (control element). One can select individual components of the expression control region that are appropriate for a given host and vector according to established molecular biology principles (Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York incorporated herein by reference). Suitable promoters include but are not limited to LTR or SV40 promoter, E.coli lac, tac or trp promoters and the phage lambda P_{L} promoter. Vectors will preferably incorporate an origin of replication as well as selection markers i.e. ampicilin resistance gene. Suitable bacterial vectors include pET, pQE70, pQE60, pQE-9, pbs, pD10 phagescript, psiX174, pbluescript SK, pbsks, pNH8A, pNH16a, pNH18A, pNH46A, ptrc99a, pKK223-3, pKK233-3, pDR540, pRIT5 and eukaryotic vectors pBlueBacIII, pWLNEO, pSV2CAT, pOG44, pXT1, pSG, pSVK3, pBPV, pMSG and pSVL. Host cells may be bacterial i.e. E.coli, Bacillus subtilis, Streptomyces; fungal i.e. Asperaillus niger, Asperaillus nidulins; yeast i.e. Saccharomyces or eukaryotic i.e. CHO, COS.

Upon expression of the polypeptide in culture, cells are typically harvested by centrifugation then disrupted by physical or chemical means (if the expressed polypeptide is not secreted into the media) and the resulting crude extract retained to isolate the polypeptide of interest. Purification of the polypeptide from culture media or lysate may be achieved by established techniques depending on the properties of the polypeptide i.e. using ammonium sulfate or ethanol precipitation , acid extraction, anion or cation exchange chromatography, phosphocellulose chromatography, hydrophobic interaction chromatography, hydroxylapatite chromatography and lectin chromatography. Final purification may be achieved using HPLC.

The polypeptide may be expressed with or without a leader or secretion sequence. In the former case the leader may be removed using post-translational processing (see US 4,431,739; US 4,425,437; and US 4,338,397 incorporated herein by reference) or be chemically removed subsequent to purifying the expressed polypeptide.

According to a further aspect, the streptococcus polypeptides of the invention may be used in a diagnostic test for streptococcus infection, in particular S. pneumoniae infection. Several diagnostic methods are possible, for example detecting streptococcus organism in a biological sample, the following procedure may be followed:
a) obtaining a biological sample from a patient;
b) incubating an antibody or fragment thereof reactive with a streptococcus polypeptide of the invention with the biological sample to form a mixture; and
c) detecting specifically bound antibody or bound fragment in the mixture which indicates the presence of streptococcus.

Alternatively, a method for the detection of antibody specific to a streptococcus antigen in a biological sample containing or suspected of containing said antibody may be performed as follows:
a) obtaining a biological sample from a patient;
b) incubating one or more streptococcus polypeptides of the invention or fragments thereof with the biological sample to form a mixture; and
c) detecting specifically bound antigen or bound fragment in the mixture which indicates the presence of antibody specific to streptococcus.

One of skill in the art will recognize that this diagnostic test may take several forms, including an immunological test such as an enzyme-linked immunosorbent assay (ELISA), a radioimmunoassay or a latex agglutination assay, essentially to determine whether antibodies specific for the protein are present in an organism.

The DNA sequences encoding polypeptides of the invention may also be used to design DNA probes for use in detecting the presence of streptococcus in a biological sample suspected of containing such bacteria. The detection method of this invention comprises:
a) obtaining the biological sample from a patient;
b) incubating one or more DNA probes having a DNA sequence encoding a polypeptide of the invention or fragments thereof with the biological sample to form a mixture; and
c) detecting specifically bound DNA probe in the mixture which indicates the presence of streptococcus bacteria.

The DNA probes of this invention may also be used for detecting circulating streptococcus i.e. S.pneumoniaenucleic acids in a sample, for example using a polymerase chain reaction, as a method of diagnosing streptococcus infections. The probe may be synthesized using conventional techniques and may be immobilized on a solid phase, or may be labelled with a detectable label. A preferred DNA probe for this application is an oligomer having a sequence complementary to at least about 6 contiguous nucleotides of the streptococcus pneumoniae polypeptides of the invention.

Another diagnostic method for the detection of streptococcus in a patient comprises:
a) labelling an antibody reactive with a polypeptide of the invention or fragment thereof with a detectable label;
b) administering the labelled antibody or labelled fragment to the patient; and
c) detecting specifically bound labelled antibody or labelled fragment in the patient which indicates the presence of streptococcus.

A further aspect of the invention is the use of the streptococcus polypeptides of the invention as immunogens for the production of specific antibodies for the diagnosis and in particular the treatment of streptococcus infection. Suitable antibodies may be determined using appropriate screening methods, for example by measuring the ability of a particular antibody to passively protect against streptococcus infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the streptococcus pneumoniae polypeptides but is preferably specific for one.

New antigens designated BVH-3, BVH-11, BVH-11-2, BVH-28 and BVH-71, truncated polypeptides comprising fragments of the new antigens designated BVH-3, BVH-11, BVH-11-2, BVH-28 and BVH-71; and chimeric polypeptides comprising fragments of the new antigens designated BVH-3, BVH-11, BVH-11-2, BVH-28 and HVH-71 are disclosed herein. The following is a reference table summarizing the relation between the antigens of the present invention:

| **Family** | **Nucleotide SEQ ID NO** | **Polypeptide SEQ ID NO** |
|---|---|---|
| BVH-3 | | |
| BVH-3 (inv) | 1, 11 | 2 |
| BVH-3A " | 7 | 8 |
| BVH-3B " | 9 | 10 |
| BVH-3 SP63 " | 15 | 16 |
| BVH-3M " | | 55 |
| BVH-3AD (ref) | | 56 |
| L-BVH-3AD " | | 57 |
| New12 " | 76 | 58 |
| BVH-3C " | | 59 |
| New1 (inv) | | 64 |
| New2 " | | 65 |
| New3 " | | 66 |
| New15 " | | 78 |

| BVH-11 | | |
|---|---|---|
| BVH-11 (ref) | 3 , 12 | 4 |
| BVH-11-2 " | 13 | 14 |
| BVH-11M " | | 60 |
| BVH-11A " | | 61 |
| BVH-11B also referred to as NEW13 " | | 62 |
| BVH-11C " | | 63 |
| New4 " | | 67 |
| New5 '' | | 68 |
| New6 " | | 69 |
| New7 " | | 70 |
| New8 " | | 71 |
| New9 " | | 72 |
| BVH-11-2M " | | 73 |
| New10 " | | 74 |
| New11 " | | 75 |
| New12 " | 76 | 58 |
| New14 " | | 77 |
| New16 " | | 79 |

| BVH-28 | | |
|---|---|---|
| BVH-28 " | 5 | 6 |

| BVH-71 | | |
|---|---|---|
| GBS " | 80 | 81 |
| GAS " | 82 | 83 |

| | | |
|---|---|---|
| Note. "inv" means of the invention "ref" means incorporated for reference | | |

### EXAMPLE 1

This example illustrates the cloning of S. pneumoniae genes.

The coding region of S. pneuinoniae gene BVH-3 **(SEQ ID NO: 1)** and the coding region of S. pneumoniae gene BVH-28 **(SEQ ID NO: 5)** were amplified by PCR (DNA Thermal Cycler GeneAmp PCR system 2400 Perkin Elmer, San Jose, CA) from genomic DNA of serogroup 6 S. pneumoniae strain SP64 using the oligos that contained base extensions for the addition of restriction sites BgIII (AGATCT) and XbaI (TCTAGA). PCR products were purified from agarose gel using a QIAquick gel extraction kit from QIAgen (Chatsworth, CA), digested BglII-XbaI (Pharmacia Canada Inc, Baie d'Urfé, Canada), extracted with phenol : chloroform and precipitated with ethanol. The Superlinker vector pSL301 (Invitrogen, San Diego, CA) was digested with BglII and XbaI and purified from agarose gel using a QIAquick gel extraction kit from QIAgen (Chatsworth, CA). The BglII-XbaI genomic DNA fragments were ligated to the Bg1II-XbaI pSL301 vector. The ligated products were transformed into E. coli strain DH5a [f80 *lac*Z DM15 *end*A1 *rec*A1 *hsd*R17 (^{r}K^{-m}K⁺) *sup*E44 *thi-*11⁻ *gyr*A96 *rel*A1 D(*lac*ZYA-*arg*F)U169] (Gibco BRL, Gaithersburg, MD) according to the method of Simanis (Hanahan, D. DNA Cloning, 1985, D.M. Glover (ed), pp. 109-135). Recombinant pSL301 plasmids (rpSL301) containing either BVH-3 or BVH-28 gene were purified using a QIAgen kit (Chatsworth, CA) and DNA inserts were confirmed by nucleotide sequence analysis (Taq Dye Deoxy Terminator Cycle Sequencing kit, ABI, Foster City, CA). Recombinant rpSL301 (rpSL301) were digested with the restriction enzymes BglII (AGATCT) and XhoI (CTCGAG). DNA fragments BglII-XhoI were purified using the QIAquick gel extraction kit from QIAgen (Chatsworth, CA). pET-32c(+) expression vector (Novagen, Madison, WI) containing the thioredoxin-His·Tag sequence was digested with BamHI (GGATCC) and XhoI and gel extracted using the QIAquick gel extraction kit from QIAgen (Chatsworth, CA). The BglII-XhoI DNA fragments were ligated to the BamHI-XhoI pET-32c(+) vector to create the coding sequence for thioredoxin-His·Tag-BVH-3 or thioredoxin-His·Tag-BVH-28 fusion protein. The ligated products were transformed into E. coli strain DH5a [f80 *lac*Z DM15 *end*A1 *rec*A1 *hsd*R17 (^{r}K^{-m}K⁺) *sup*E44 *thi*-11⁻*gyr*A96 *rel*A1 D(*lac*ZYA-*arg*F)U169] (Gibco BRL, Gaithersburg, MD) according to the method of Simanis (Hanahan, D. DNA Cloning, 1985, D.M. Glover (ed), pp. 109-135). Recombinant pET-32c(+) plasmids were purified using a QIAgen kit (Chatsworth, CA) and the nucleotide sequences at the fusion sites of thioredoxin-His·Tag and DNA insert were verified by DNA sequencing (Taq Dye Deoxy Terminator Cycle Sequencing kit, ABI, Foster City, CA).

### EXAMPLE 2

This example illustrates the cloning of S. pneumoniae protein genes in CMV plasmid pCMV-GH.

The DNA coding region of a S. pneumoniae protein was inserted in phase downstream of a human growth hormone (hGH) gene which was under the transcriptional control of the cytomegalavirus (CMV) promotor in the plasmid vector pCMV-GH (Tang et al., Nature, 1992, 356 :152). The CMV promotor is non functional plasmid in E. coli cells but active upon administration of the plasmid in eukaryotic cells. The vector also incorporated the ampicillin resistance gene.

The coding region of BVH-3 gene (**SEQ ID NO: 1**) and BVH-28 gene (**SEQ ID NO: 5**) were obtained from rpSL301 (see example 1) using restriction enzymes BglII (AGATCT) and XbaI (TCTAGA). The digested products were purified from agarose gel using the QIAquick gel extraction kit from QIAgen (Chatsworth, CA). The pCMV-GH vector (Laboratory of Dr. Stephen A. Johnston, Department of Biochemistry, The University of Texas, Dallas, Texas) containing the human growth hormone to create fusion proteins was digested with BglII and XbaI and purified from agarose gel using the QIAquick gel extraction kit from QIAgen (Chatsworth, CA). The BglII-XbaI DNA fragments were ligated to the BglII-XbaI pCMV-GH vector to create the hGH-BVH-3 or hGH-BVH-28 fusion protein under the control of the CMV promoter. The ligated products were transformed into E. coli strain DH5a[f80 *lac*Z DM15 *end*A1 *rec*A1 *hsd*R17 (^{r}K^{-m}K⁺) *sup*E44 *thi*-11⁻ gyrA96 *rel*A1 D(*lac*ZYA-*arg*F)U169] (Gibco BRL, Gaithersburg, MD) according to the method of Simanis (Hanahan, D. DNA Cloning, 1985, D.M. Glover (ed), pp. 109-135). The recombinant pCMV plasmids were purified using a QIAgen kit (QIAgen, Chatsworth, CA).

The coding region of BVH-11 gene (**SEQ ID NO: 3**) was amplified by PCR (DNA Thermal Cycler GeneAmp PCR system 2400 Perkin Elmer, San Jose, CA) from genomic DNA of serogroup 6 S. pneumoniae strain SP64 using the oligos that contained base extensions for the addition of restriction sites BglII (AGATCT) and HindIII (AAGCTT). The PCR product was purified from agarose gel using a QIAquick gel extraction kit from QIAgen (Chatsworth, CA), digested with restriction enzymes (Pharmacia Canada Inc, Baie d'Urfe, Canada), extracted with phenol : chloroform and precipitated with ethanol. The pCMV-GH vector (Laboratory of Dr. Stephen A. Johnston, Department of Biochemistry, The University of Texas, Dallas, Texas) was digested with BglII and HindIII and purified from agarose gel using the QIAquick gel extraction kit from QIAgen (Chatsworth, CA). The BglII-HindIII DNA fragment was ligated to the BglII-HindIII pCMV-GH vector to create the hGH-BVH-11 fusion protein under the control of the CMV promoter. The ligated products were transformed into E. coli strain DH5a[f80 *lac*Z DM15 *end*A1 *rec*A1 *hsd*R17 (^{r}K^{-m}K⁺) *sup*E44 *thi*-11⁻ *gyr*A96 *rel*A1 D(*lac*ZYA-*arg*F)U169] (Gibco BRL, Gaithersburg, MD) according to the method of Simanis (Hanahan, D. DNA Cloning, 1985, D.M. Glover (ed), pp. 109-135). The recombinant pCMV plasmid was purified using a QIAgen kit (Chatsworth, CA) and the nucleotide sequence of the DNA insert was verified by DNA sequencing.

### EXAMPLE 3

This example illustrates the use of DNA to elicit an immune response to S. pneumoniae antigens.

A group of 8 female BALB/c mice (Charles River, St-Constant, Quebec, Canada) were immunized by intramuscular injection of 50 µl three times at two- or three-week intervals with 100 µg of recombinant pCMV-GH encoding the BVH-3, BVH-11 or the BVH-28 gene in presence of 50 µg of granulocyte-macrophage colony-stimulating factor (GM-CSF)- expressing plasmid pCMV-GH-GM-CSF (Laboratory of Dr. Stephen A. Johnston, Department of Biochemistry, The University of Texas, Dallas, Texas). As control, a group of mice were injected with 100 µg of pCMV-GH in presence of 50 µg of pCMV-GH-GM-CSF. Blood samples were collected from the orbital prior to each immunization and seven days following the third injection and serum antibody responses were determined by ELISA using thioredoxin-His·Tag-S. pneumoniae fusion protein as coating antigen. DNA immunization with recombinant plasmid pCMV-GH encoding the BVH-3, BVH-11 or the BVH-28 S. pneumoniae protein induced antibody reactive against the respective recombinant protein. The reciprocal antibody titers, defined as the highest serum dilution at which the absorbance values were 0.1 above the background values, were above 4x10³.

### EXAMPLE 4

This example illustrates the production and purification of recombinant S. pneumoniae proteins.

The recombinant pET plasmids containing the BVH-3, BVH-11 or the BVH-28 gene corresponding to the **SEQ ID NO: 1, SEQ ID NO: 3** or the **SEQ ID NO: 5** respectively were transformed by electroporation (Gene Pulser II apparatus, BIO-RAD Labs, Mississauga, Canada) into E. coli strain AD494 (DE3) (D*ara*⁻leu7697 D*lac*X74 D*phoA* PvuII *pho*R DmalF3 F'[*lac⁺*(*lac*I*^{q}*) *pro*] trxB::Kan) (Novagen, Madison, WI). In this strain of E. coli, the T7 promotor controlling expression of the fusion protein is specifically recognized by the T7 RNA polymerase (present on the 1DE3 prophage) whose gene is under the control of the lac promotor which is inducible by isopropyl-β-d-thio-galactopyranoside (IPTG). The transformant AD494(DE3)/rpET was grown at 37°C with agitation at 250 rpm in LB broth (peptone 10g/L, yeast extract 5g/L, NaCl 10g/L) containing 100µg of ampicillin (Sigma-Aldrich Canada Ltd., Oakville, Canada) per ml until the A₆₀₀ reached a value of 0.6. In order to induce the production of the thioredoxin-His·Tag-BVH-3, thioredoxin-His·Tag-BVH-11 or thioredoxin-His·Tag-BVH-28 fusion protein, the cells were incubated for 2 additional hours in the presence of IPTG at a final concentration of 1 mM. Induced cells from a 100 ml culture were pelleted by centrifugation and frozen at -70°C.

The purification of the fusion proteins from the soluble cytoplasmic fraction of IPTG-induced AD494(DE3)/rpET was done by affinity chromatography based on the properties of the His·Tag sequence (6 consecutive histidine residues) to bind to divalent cations (Ni²⁺) immobilized on the His-Bind metal chelation resin. Briefly, the pelleted cells obtained from a 100mL culture induced with IPTG were resuspended in phosphate-buffered (PBS):500mM NaCl pH7.1, sonicated and spun at 20,000 X g for 20 min to remove debris. The supernatant was filtered (0.22µm pore size membrane) and deposited on a HiTrap® 1mL chelating pre-packed ready-to-use column (Pharmacia Biotech, Baie d'Urfé, Canada). The thioredoxin-His·Tag-S. pneumoniae fusion protein was eluted with 1M imidazole-500mM NaCl-PBS pH7.1. The removal of the salt and imidazole from the sample was done by dialysis against PBS at 4°C. The quantities of fusion protein obtained from the soluble fraction of E. coli was estimated by MicroBCA (Pierce, Rockford, Illinois).

### EXAMPLE 5

This example illustrates the protection of mice against fatal pneumococcal infection by immunization.

Groups of 8 female BALB/c mice (Charles River) were immunized subcutaneously three times at three-week intervals with either 25 µg of affinity purified thioredoxin-His·Tag-BVH-3 fusion protein in presence of 15 µg of QuilA adjuvant (Cedarlane Laboratories Ltd, Hornby, Canada) or, as control, with QuilA adjuvant alone in PBS. Blood samples were collected from the orbital sinus on day 1, 22 and 43 prior to each immunization and seven days (day 50) following the third injection. One week later the mice were challenged with approximately 10⁶ CFU of the type 3 S. pneumoniae strain WU2. Samples of the S. pneumoniae challenge inoculum were plated on chocolate agar plates to determine the CFU and to verify the challenge dose. Deaths were recorded for a period of 14 days and on day 14 post-challenge, the surviving mice were sacrificied and blood samples tested for the presence of S. pneumoniae organisms. The survival data are shown in table 1.

Prechallenge sera were analyzed for the presence of antibodies reactive with S. pneumoniae by standard immunoassays. Elisa and immunoblot analyses indicated that immunization with recombinant S. pneumoniae protein produced in E. coli elicited antibodies reactive with both, recombinant and native pneumococcal protein.

**Table 1. Protection mediated by recombinant BVH-3 protein**

| Immunogen | No. of mice alive : no. of mice dead | Median day of death |
|---|---|---|
| | 14 days post-challenge | |
| BVH-3 | 8 : 0 | >14 |
| none | 0 : 8 | 1 |

All mice immunized with BVH-3 recombinant protein survived to infection while none of the control mice given adjuvant alone survived. There was a significant difference in survival between the two groups of mice (P<0.0001, log rank test for nonparametric analysis of survival curves; P=0.0002, Fisher's exact test). All hemocultures from surviving mice were negative at day 14 post-challenge.

### EXAMPLE 6

This example describes the cloning of BVH-3 and BVH-11 genes from a variety of S. pneumoniae strains and the molecular conservation of these genes.

Molecular analysis of chromosomal DNA from various S. pneumoniae isolates with DNA probes spanning different regions of BVH-3 or BVH-11 revealed the presence of one BVH-3 gene copy and two BVH-11 gene copies. The two BVH-11 gene copies are not identical and the genes were arbitrarily designated BVH-11 (SEQ ID NO:12; ORF at nucleotides 45 to 2567) and BVH-11-2 (SEQ ID NO:13; ORF at nucleotides 114 to 2630).

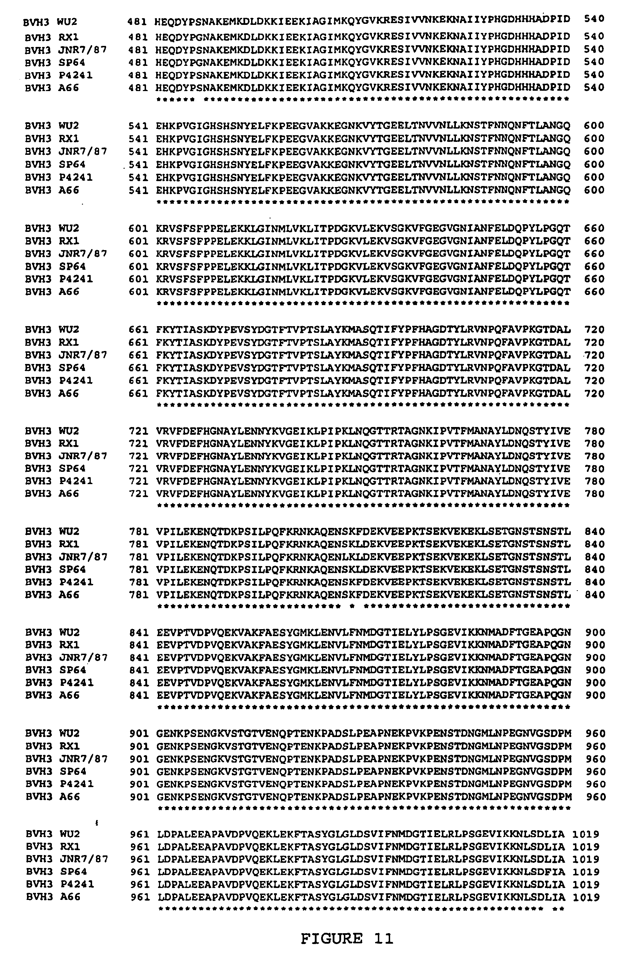

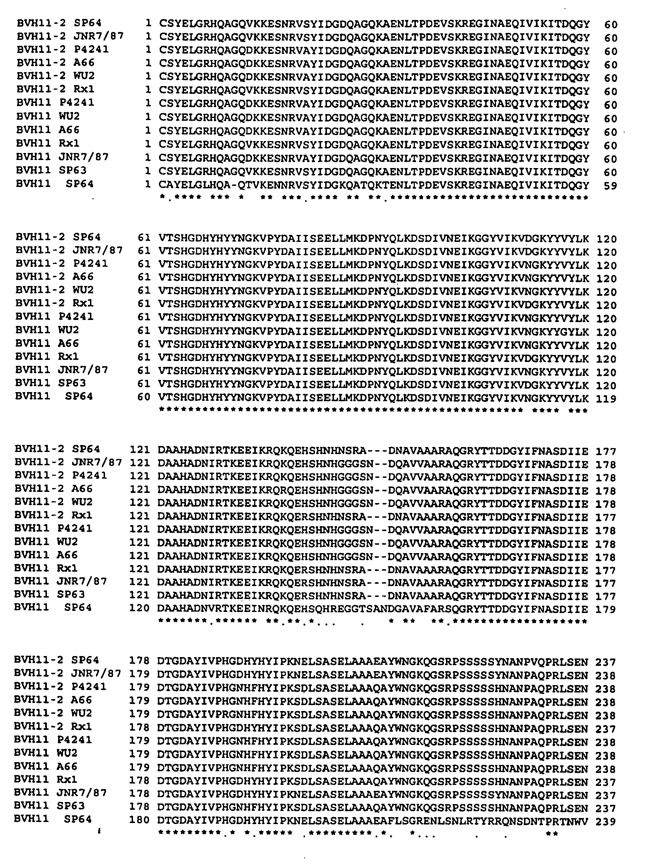

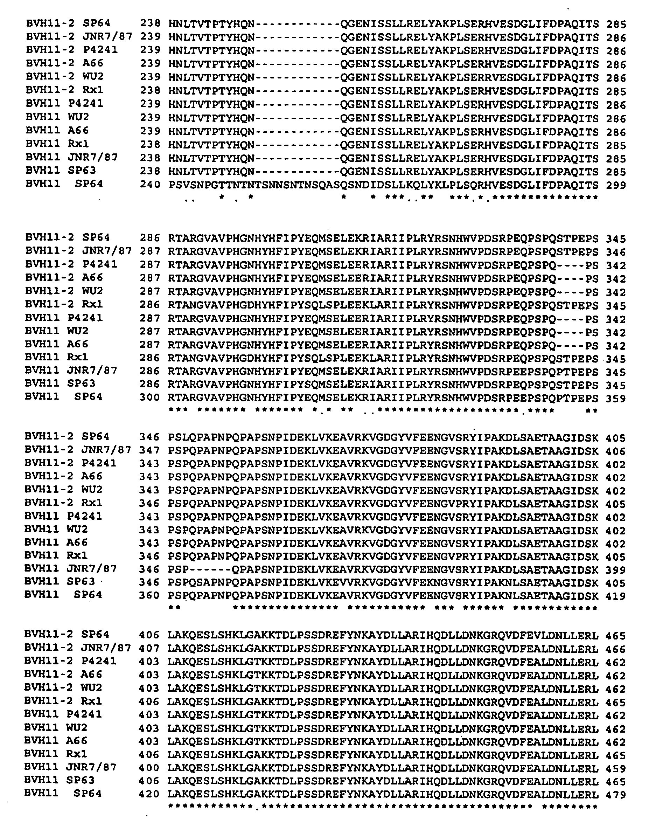

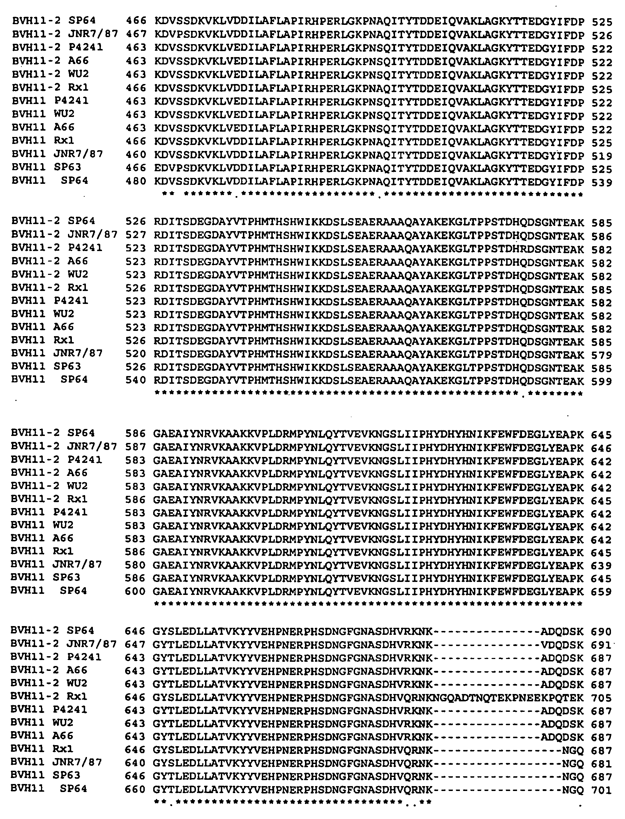

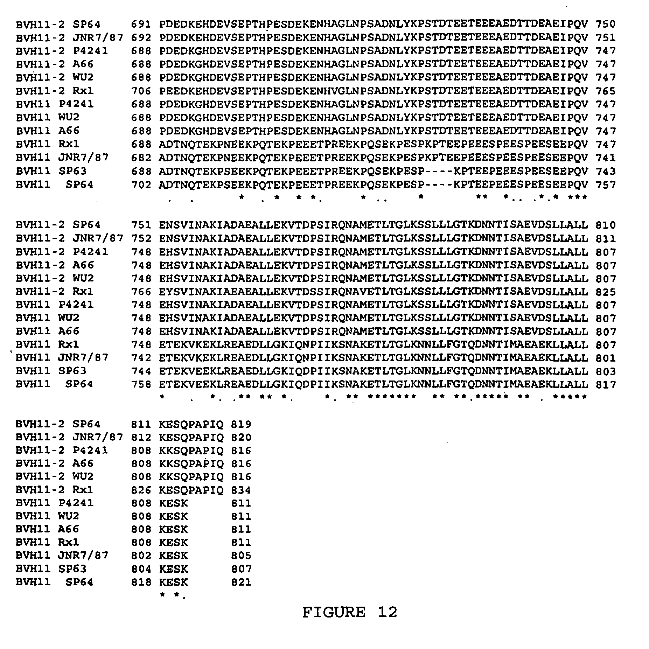

The first amino acids of the BVH-3 and BVH-11 coding regions have the characteristics of leader sequences also known as signal peptides. The consensus signal peptidase cleavage site L-X-X-C of lipoprotein modification/processing sites was present in the sequences. Mature BVH-3, BVH-11 and BVH-11-2 proteins from S. pneumoniae SP64 have 1019, 821 and 819 amino acids, respectively. The regions of S. pneumoniae genes coding for mature BVH-3, termed BVH-3M, (nucleotides 1837 - 4896; SEQ. ID. NO: 11), BVH-11M (nucleotides 102-2567; SEQ. ID. NO: 12) and BVH-11-2M (nucleotides 171-2630; SEQ. ID. NO: 13), were amplified by PCR(DNA Thermal Cycler GeneAmp PCR system 2400 Perkin Elmer, San Jose, CA) from genomic DNA of 6 or 7 S. pneumoniae strains. Serogroup 6 S. pneumoniae SP64 and serogroup 9 SP63 clinical isolates were provided by the laboratoire de la santé publique du Québec, Sainte-Anne-de-Bellevue; serotype 4 strain JNR.7/87 was provided by Andrew Camilli, Tufts University School of Medicine, Boston; Rx1 strain, a nonencapsulated derivative of the type 2 strain D39 and the type 3 strains A66 and WU2 were provided by David E. Briles from University of Alabama, Birmingham and the type 3 clinical isolate P4241 was provided by the centre de recherche en infectiologie du centre hospitalier de l'université Laval, Sainte-Foy. The sets of oligonucleotide primers OCRR479-OCRR480; HAMJ160-OCRR488 and HAMJ160-HAMJ186, that contained base extensions for the addition of restriction sites were used for the amplification of BVH-3, BVH-11 and BVH-11-2 gene, respectively, with the exception of BVH-11 gene from SP64 strain which was amplified using the set of primers consisting of HAMJ487 and OCRR488. Primer sequences are listed below (Table 2). PCR products were purified from agarose gel using a QIAquick gel extraction kit from QIAgen (Chatsworth, CA) and digested BglII-XbaI or BglII-HindIII (Pharmacia Canada Inc, Baie d'Urfé, Canada). Digestions were cleaned using a QIAquick PCR purification kit from QIAgen (Chatsworth, CA). The PCR products were ligated to the BglII-XbaI or BglII-HindIII pSL301 vector. The ligated products were transformed into E. coli strain DH5α [φ80 *lac*Z ΔM15 *end*A1 *rec*A1 *hsd*R17 (^{r}K^{-m}K⁺) *sup*E44 *thi*-1λ⁻ gyrA96 *rel*A1 Δ(*lac*ZYA-*arg*F)U169] (Gibco BRL, Gaithersburg, MD) according to the method of Simanis (Hanahan, D. DNA Cloning, 1985, D.M. Glover (ed), pp. 109-135). Recombinant pSL301 plasmids (rpSL301) containing BVH-3, BVH-11 or BVH11-2 were purified using a QIAgen kit (Chatsworth, CA) and DNA inserts were sequenced (Taq Dye Deoxy Terminator Cycle Sequencing kit, ABI, Foster City, CA). The figures 11 and 12 depict the consensus sequence established from the BVH-3, and BVH-11 deduced amino acid sequences, respectively. Comparison of BVH-3 protein sequences revealed 99 to 100% identity of sequences for all strains with the exception that BVH-3 from serogroup 9 SP63 strain (SEQ. ID. NO: 15 and SEQ. ID. NO: 16) misses a stretch of 177 amino acids corresponding to residues 244 to 420 on BVH-3' protein sequence of S. pneumoniae SP64. Analysis of sequences of additional serogroup 9 strains revealed BVH-3 molecule having the same deletion in 3 out of 4 strains thus suggesting that the 3 strains are members of a S. pneumoniae serogroup 9 clone.

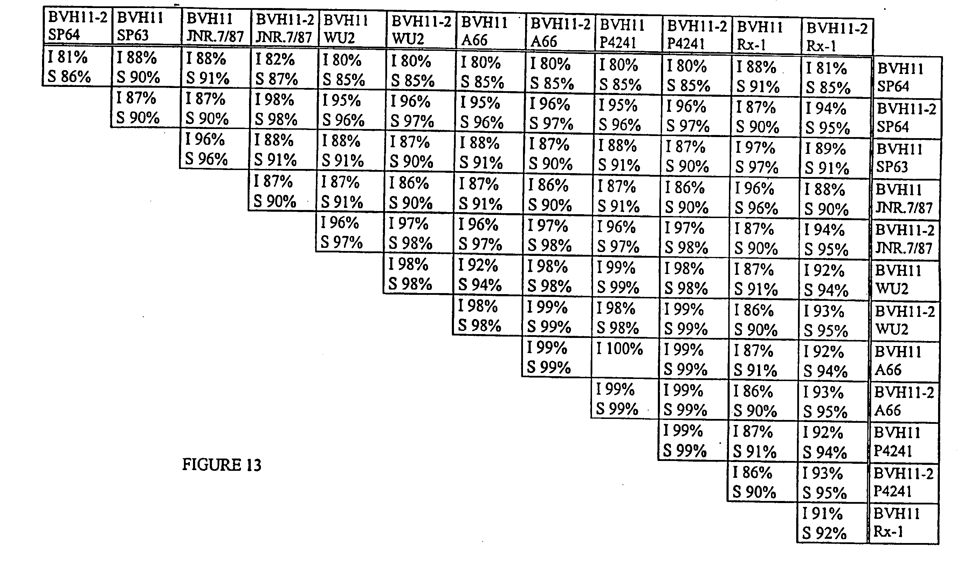

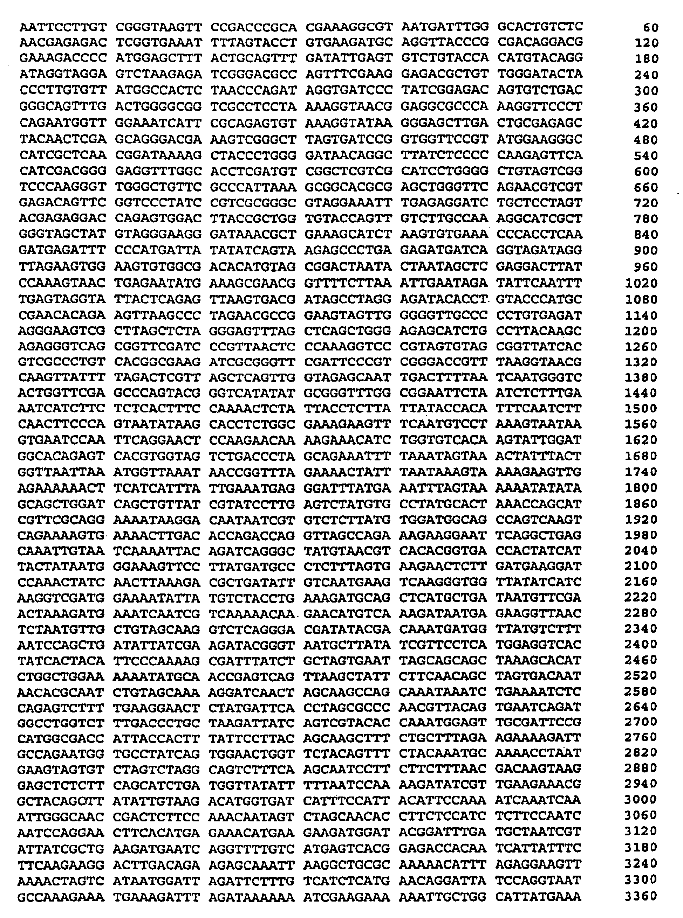

Comparison of 13 BVH-11 nucleotide sequences obtained from 7 S. pneumoniae strains, revealed that the nucleotide sequences are very similar. Computer analysis (MacVector, Clustal W 1.4) using multiple alignment of the predicted BVH-11 protein sequences revealed that these sequences were 75% identical and 82 % homologous on a length of 834 amino acids. Pairwise alignment revealed 80 to 100% identity (Figure 13). The sequences showed great similarity in overall organization. Variability in the primary sequence of these proteins is almost restricted to the last 125 amino acids in the C-terminal portion of the proteins. This region constitutes a domain. Close examination of this domain revealed two groups of sequences. The first 9 sequences from the figure 13 belong to one group while the last 4 sequences belong to another group. A 39% identity value is obtained when the domain sequences of the 13 proteins are compared (MacVector, Clustal W 1.4). The identity value increased to more than 92% when sequences belonging to a same group are compared.

### EXAMPLE 7

This example illustrates the homology of portions of BVH-3 and BVH-11 genes.

Molecular analysis with DNA probes derived from BVH-3 and BVH-11 genes indicated that BVH-3 and BVH-11 were related. In dot blot hybridization studies, DNA probe consisting of either, BVH-3 or BVH-11, gene sequence hybridized to both, BVH-3 and BVH-11 genes thus indicating that BVH-3 and BVH-11 genes shared homologous sequences. Comparison of sequences revealed that the ORFs and the proteins were 43 and 33% identical, respectively. Closer examination revealed that the region corresponding to amino acids 1 to 225 in BVH-3 and 1 to 228 in BVH-11 were 73 and 75% identical at the DNA and protein level, respectively. In contrast, the 3' regions corresponding to amino acids 226 to 1039 from BVH-3 and amino acids 229-840 from BVH-11 were only 34 and 22% identical at the DNA and protein level, respectively. Thus the 5' termini of BVH-3 and BVH-11 genes appear to contain highly conserved sequences while the remaining parts of the genes are highly divergent. These results suggest that BVH-3 and BVH-11 might share similar functions mediated by sequences present in the conserved region whereas BVH-3- and BVH-11-specific functions might be mediated by sequences in the divergent region.

### EXAMPLE 8

This example describes the cloning of truncated BVH-3, BVH-11 and BVH-11-2 genes by polymerase chain reaction (PCR) and the expression of truncated BVH-3 and BVH-11 molecules.

Gene fragments were amplified by PCR using pairs of oligonucleotide engineered to amplify fragments spanning the BVH-3 **(SEQ ID NO: 1** and **SEQ ID NO: 11),** BVH-11 **(SEQ ID NO: 3** and **SEQ ID NO: 12)** or BVH-11-2 **(SEQ ID NO: 13)** gene from S. pneumoniae strain SP64. Each of the primers had a restriction endonuclease site at the 5' end, thereby allowing directional in-frame cloning of the amplified product into the digested plasmid vector (Tables 2 and 3). PCR-amplified products were digested with restriction endonucleases and ligated to either linearized plasmid pSL301 (see example 1), pCMV-GH (see example 2) or pET (Novagen, Madison, WI) expression vector digested likewise or digested with enzymes that produce compatible cohesive ends. Recombinant pSL301 and recombinant pCMV-GH plasmids were digested with restriction enzymes for the in-frame cloning in pET expression vector. Clones were first stabilized in E. coli DH5α before introduction into E. coli

BL21(λDE3) or AD494 (λDE3) for expression of truncated BVH-3 or BVH-11 molecules. Each of the resultant plasmid constructs was confirmed by nucleotide sequence analysis. The recombinant proteins were expressed as N-terminal fusions with the thioredoxin and His-tag or as C-terminal fusions with an His-tag. The expressed recombinant proteins were purified from supernatant fractions obtained from centrifugation of sonicated IPTG-induced E. coli cultures using a His-Bind metal chelation resin (QIAgen, Chatsworth, CA). The gene products generated are listed in the table 3. The gene products corresponding to the N-terminal region including the signal sequence are designated as Lipidated-proteins or lipoproteins (L-proteins). The gene products corresponding to the N-terminal region lacking the signal sequence are identified as protein without signal sequence (w/o ss).

**Table 2. List of PCR oligonucleotide primers**

| Primer | SEQ. ID. | Sequence 5' - 3' | Nucleotide position | Restriction sites |
|---|---|---|---|---|
| OCRR 479 | 17 | cagtagatctgtgcctatgcactaaac | SEQ ID 1 :61-78 | BgIII |
| OCRR 480 | 18 | gatctctagactactgctattccttacgctatg | SEQ ID 11 :4909-4887 | XbaI |
| OCRR 497 | 19 | atcactcgagcattacctggataatcctgt | SEQ ID 1:1525-1506 | XhoI |
| OCRR 498 | 20 | ctgctaagcttatgaaagatttagat | SEQ ID 1 : 1534-1548 | HindIII |
| OCRR 499 | 21 | gatactcgagctgctattccttac | SEQ ID 11:4906-4893 | XhoI |
| HAMJ 172 | 22 | gaatctcgagttaagctgctgctaattc | SEQ ID 1 : 675-661 | XhoI |
| HAMJ 247 | 23 | gacgctcgagcgctatgaaatcagataaattc | SEQ ID 1 :3117-3096 | XhoI |
| HAMJ 248 | 24 | gacgctcgagggcattacctggataatcctgttcatg | SEQ BD 1 :1527-1501 | XhoI |
| HAMJ 249 | 25 | cagtagatctcttcatcatttattgaaaagagg | SEQ ID 11 : 1749-1771 | BgIII |
| HAMJ 278 | 26 | ttatttcttccatatggacttgacagaagagcaaattaag | SEQ ID 1:1414-1437 | NdeI |
| HAMJ 279 | 27 | cgccaagcttcgctatgaaatcagataaattc | SEQ ID 1 :3117-3096 | HindIII |
| HAMJ 280 | 28 | cgccaagcttttccacaatataagtcgattgatt | SEQ ID 1 :2400-2377 | HindIII |
| HAMJ 281 | 29 | ttatttcttccatatggaagtacctatcttggaaaaagaa | SEQ ID 1 :2398-2421 | Ndel |
| HAMJ 300 | 30 | ttatttcttccatatggtgcctatgcactaaaccagc | SEQ ID 1 :62-82 | NdeI |
| HAMJ 313 | 31 | ataagaatgcggccgcttccacaatataagtcgattgatt | SEQ ID 1 :2400-2377 | NotI |
| OCRR 487 | 32 | cagtagatctgtgcttatgaactaggtttgc | SEQ ID 3 :58-79 | BglII |
| OCRR 488 | 33 | gatcaagcttgctgctacctttacttactctc | SEQ ID 12 :2577-2556 | HindIII |
| HAMJ 171 | 34 | ctgagatatccgttatcgttcaaacc | SEQ ID 3 :1060-1075 | EcoRV |
| HAMJ 251 | 35 | ctgcaagcttttaaaggggaataatacg | SEQ ID 3 :1059-1045 | HindIII |
| HAMJ 264 | 36 | cagtagatctgcagaagccttcctatctg | SEQ ID 3 :682-700 | BglII |
| HAMJ 282 | 37 | tcgccaagcttcgttatcgttcaaaccattggg | SEQ ID 3:1060-1081 | HindIII |
| HAMJ 283 | 38 | ataagaatgcggccgccttactctcctttaataaagccaat agtt | SEQ ID 3 :2520-2492 | NdeI |
| HAMJ 284 | 39 | catgccatggacattgatagtctcttgaaacagc | SEQ ID 3 :856-880 | NcoI |
| HAMJ 285 | 40 | cgccaagcttcttactctcctttaataaagccaatag | SEQ ID 3 :2520-2494 | HindIII |
| HAMJ 286 | 41 | cgacaagcttaacatggtcgctagcgttacc | SEQ ID 3:2139-2119 | HindIII |
| HAMJ 287 | 42 | cataccatgggcctttatgaggcacctaag | SEQ ID 3 :2014-2034 | NcoI |
| HAMJ 288 | 43 | cgacaagcttaagtaaatcttcagcctctctcag | SEQ ID 3:2376-2353 | HindIII |
| HAMJ 289 | 44 | gataccatggctagcgaccatgttcaaagaa | SEQ ID 3:2125-2146 | NcoI |
| HAMJ 290 | 45 | cgccaagcttatcatccactaacttgactttatcac | SEQID 3:1533-1508 | HindIII |
| HAMJ 291 | 46 | cataccatggatattcttgccttcttagctccg | SEQ ID 3:1531-1554 | NcoI |
| HAMJ 301 | 47 | catgccatggtgcttatgaactaggtttgc | SEQ ID 3 :59-79 | NcoI |
| HAMJ 302 | 48 | cgccaagctttagcgttaccaaaaccattatc | SEQ ID 3 :2128-2107 | HindIII |
| HAMJ.160 | 49 | gtattagatctgttcctatgaacttggtcgtcacca | SEQ ID 13 : 172-196 | BglII |
| HAMJ 186 | 50 | cgcctctagactactgtataggagccgg | SEQ ID 13: 2460-2443 | XbaI |
| HAMJ 292 | 51 | catgccatggaaaacatttcaagccttttacgtg | SEQ ID 11: 754-778 | NcoI |
| HAMJ 293 | 52 | cgacaagcttctgtataggagccggttgactttc | SEQ ID 11: 2457-2434 | HindIII |
| HAMJ 294 | 53 | catgccatggttcgtaaaaataaggcagaccaag | SEQ ID 11 : 2038-2062 | Ncol |
| HAMJ 297 | 54 | catgccatggaagcctattggaatgggaag | SEQ ID 11 : 622-642 | NcoI |

**Table 3. Lists of truncated BVH-3 and BVH-11 gene products generated from S. pneumoniae SP64**

| PCR-primer sets - | Protein designation | Identification (encoded amino acids) | SEQ. ID.NO. | Cloning vector |
|---|---|---|---|---|
| OCRR479-OCRR480 | BVH-3M | BVH-3 w/o ss (21-1039) | 55 | pSL301 |
| OCRR479-OCRR497 | BVH-3AD | BVH-3 N' end w/o ss (21-509) | 56 | pSL301 |
| HAMJ248-HAMJ249 | L-BVH-3AD | BVH-3 N'end (1-509) | 57 | pET-21(+) |
| OCRR498-OCRR499 | BVH-3B | BVH-3 C'end (512-1039) | 10 | pSL301 |
| OCRR479-HAMJ172 | BVH-3C | BVH-3 N' end w/o ss (21-225) | 59 | pET-32 c(+) |
| OCRR487-OCRR488 | BVH-11M | BVH-11 w/o ss (20-840) | 60 | pCMV-GH |
| HAMJ251-OCRR487 | BVH-11A | BVH-11 N' end w/o ss (20-353) | 61 | pET-32 c (+) |
| HAMJ171-OCRR488 | BVH-11B | BVH-11 C'end (354-840) | 62 | pET-32 a(+) |
| HAMJ264-OCRR488 | BVH-11C | BVH-11 C'end (228-840) | 63 | pET-32a(+) |
| HAMJ278-HAMJ279 | NEW1 | BVH-3 C'end (472-1039) | 64 | pET-21b(+) |
| HAMJ278-HAMJ280 | NEW2 | BVH-3 C'end (472-800) | 65 | pET-21b(+) |
| HAMJ281-HAMJ279 | NEW3 | BVH-3 C'end (800-1039) | 66 | pET-21b(+) |
| HAMJ284-HAMJ285 | NEW4 | BVH-11 C'end (286-840) | 67 | pET-21d(+) |
| HAMJ284-HAMJ286 | NEW5 | BVH-11 internal (286-713) | 68 | pET-21d(+) |
| HAMJ287-HAMJ288 | NEW6 | BVH-11 internal (672-792) | 69 | pET-21d(+) |
| HAMJ285-HAMJ289 | NEW7 | BVH-11 internal (709-840) | 70 | pET-21d(+) |
| HAMJ284-HAMJ290 | NEW8 | BVH-11 internal (286-511) | 71 | pET-21d(+) |
| HAMJ286-HAMJ291 | NEW9 | BVH-11 internal (511-713) | 72 | pET-21d(+) |
| HAMJ160-HAMJ186 | BVH-11-2M | BVH-11-2 w/o ss (20-838) | 73 | pSL301 |
| HAMJ292-HAMJ293 | NEW10 | BVH-11-2 C'end (271-838) | 74 | pET-21d(+) |
| HAMJ293-HAMJ294 | NEW11 | BVH-11-2 C'end (699-838) | 75 | pET-21d(+) |
| HAMJ282-HAMJ283 | BVH-11B | BVH-11 C'end (354-840) | 62 | pET-21b(+) |
| HAMJ286-HAMJ297 | NEW14 | BVH-11-2 internal (227-699) | 77 | pET-21d(+) |
| HAMJ300-HAMJ313 | NEW15 | BVH-3 N' end w/o ss (21-800) | 78 | pET-21b(+) |
| HAMJ301-HAMJ302 | NEW16 | BVH-11 N'end w/o ss (20-709) | 79 | pET-21d(+) |

### EXAMPLE 9

This example describes the isolation of monoclonal antibodies (Mabs) and the use of Mabs to characterize BVH-3, BVH-11 and BVH-11-2 protein epitopes.

Female BALB/c mice (Charles River) were immunized subcutaneously with BVH-3, BVH-11 or BVH-11-2 gene products from S. pneumoniae strain SP64 in presence of 15 *µ*g of QuilA adjuvant (Cedarlane Laboratories Ltd, Hornby, Canada). One set of mice (fusion experiment 1) were immunized on day 1 and 14 with 25 µg of affinity purified thioredoxin-His•Tag-BVH-3M fusion protein. A second group of mice (fusion experiment 2) were immunized three times at three-week intervals with 25 µg of affinity purified thioredoxin-His•Tag-BVH-11M. A third group of mice (fusion experiment 3) were immunized on day 1 and day 15 with 25 µg of affinity purified thioredoxin-His•Tag-BVH-11-2M fusion protein. A fourth group of mice (fusion experiment 4) were immunized on day 1 with 25 µg of affinity purified thioredoxin-His•BVH-11B fusion protein and boosted by intravenous injection on day 16 and on day 37 with recombinant BVH-11B in PBS. Three to four days before fusion, mice were injected intravenously with 25 µg of the respective antigen suspended in PBS alone. Hybridomas were produced by fusion of spleen cells with nonsecreting SP2/0 myeloma cells as previously described by J. Hamel et al. [J. Med. Microbiol., 23, pp163-170 (1987)]. Culture supernatants of hybridomas were initially screened by enzyme-linked-immunoassay according to the procedure described by Hamel et al. (Supra) using plates coated with preparations of purified recombinant proteins or suspensions of heat-killed S. pneumoniae cells. Positive hybridomas selected on the basis of ELISA reactivity with a variety of antigens were then cloned by limiting dilutions, expanded and frozen.

Hybridomas were tested by ELISA or Western immunoblotting against BVH-3 and BVH-11 gene products in order to characterize the epitopes recognized by the Mabs. BVH-3 and BVH-11 shared common epitopes with 6 Mabs (H3-1-F9, H3-1-D4, H3-1-H12, H11-1-E7, H11-1-H10 and H11-1.1-G11) showing reactivities with both proteins (Table 4). BVH-11 and BVH-11-2 molecules from S. pneumoniae SP64 shared common epitopes not present on BVH-3 with Mabs (3A1, 13C11, 10H10, 1D8, 10G9, 10A2, 3E8, 10D7, 2H7 and 6H7) reactive with both, BVH-11 and BVH-11-2, recombinant proteins (Table 5) .

**Table 4. Reactivity of BVH-3-immunoreactive Mabs with a panel of BVH-3 and BVH-11 gene products**

| MAbs | a. Immunoreactivity with | | | | | | |
|---|---|---|---|---|---|---|---|
| | BVH-3M 21-1039 | BVH-3A 21-509 | BVH-3B 512-1039 | BVH-3C 21-225 | NEW2 472-800 | NEW3 800-1039 | BVH-11M 20-840 |
| H3-1-F9 | + | + | - | + | - | - | + |
| H3-1-D4 | + | + | - | + | - | - | + |
| H3-1-H12 | + | + | - | + | - | - | + |
| H3-2-G2 | + | + | - | - | - | - | - |
| H3-3-A1 | + | + | - | - | - | - | - |
| H3-4-D3 | + | - | + | - | - | + | - |
| H11-1-E7 | + | + | - | + | - | - | + |
| H11-1-H10 | + | + | - | + | - | - | + |
| H11-1.1-G11 | + | + | - | + ' | + | - | + |

**Table 5. Reactivity of Mabs raised against BVH-11-2 protein from S. pneumoniae strain SP64 with a panel of BVH-11 gene products**

| | b.Immunoreactivity with | | | | | | | |
|---|---|---|---|---|---|---|---|---|
| Mabs^{a} | c.BVH-11 products | | | | d.BVH-11-2 products | | | |
| | BVH-11M 20-840 | NEW8 286-511 | NEW9 511-713 | BVH-11B 354-840 | BVH-11-2 20-838 | NEW10 271-838 | NEW11 699-838 | NEW14 227-699 |
| 3A1 | + | + | - | + | + | + | - | + |
| 13C1 | + | + | + | + | + | + | - | + |
| 10H10 | + | + | + | + | + | + | - | + |
| 1D8 | + | + | - | + | + | + | - | + |
| 10G9 | + | - | - | + | + | + | - | + |
| 10A2 | + | - | - | + | + | + | - | + |
| 3E8 | + | - | - | + | + | + | - | + |
| 10D7 | + | - | - | + | + | + | - | + |
| 2H7 | + | - | - | - | + | - | - | - |
| 6H7 | + | - | - | - | + | - | - | - |
| 3A4 | - | - | - | - | + | + | + | - |
| 14H6 | - | - | - | - | + | + | + | - |
| 7G2 | - | - | - | - | + | + | - | + |
| 13R10 | - | - | - | - | + | - | - | + |
| 7E8 | - | - | - | - | + | - | - | - |
| 7H6 | - | - | - | - | + | - | - | - |

| | | | | | | | | |
|---|---|---|---|---|---|---|---|---|
| ^{a} Mabs listed in this table were not reactive with recombinant BVH-3 molecule | | | | | | | | |

The results obtained from the immunoreactivity studies of the Mabs (Table 4 and Table 5) are in agreement with the protein sequences derived from the respective gene sequences. Indeed the Mabs cross-reactive with BVH-3 and BVH-11 molecules recognized BVH-3C protein corresponding to the conserved region, and BVH-11 and BVH-11-2 specific Mabs were reactive with epitopes located on variable parts of these molecules. BVH-3 and BVH-11, and BVH-11 and BVH-11-2 can be distinguished by their reactivity with Mabs.

### EXAMPLE 10

This example illustrates the simultaneous expression of BVH-3 and BVH-11 gene products by S. pneumoniae.

A standard Western blot technique was used to investigate whether BVH-3 and BVH-11 genes were expressed in S. pneumoniae. S. pneumoniae strain SP64 and SP63 were grown overnight at 37°C in 5% CO₂ on chocolate agar plates, bacteria were suspended in PBS and heat-killed at 56°C for 20 min. For the preparation of antigens, suspensions of S. pneumoniae were treated with sample buffer containing SDS and 2-mercaptoethanol for 5 min at 100°C. Pneumococcal protein antigens were resolved by SDS-PAGE electrophoresis according to the method of Laemmli [Nature, 227, pp. 680-685 (1970)]. After SDS-PAGE, the proteins were transferred electrophoretically from the gel to nitrocellulose paper by the method of Towbin [Proc. Natl. Acad. Sci. USA, 76, pp. 4350-4354 (1979)] and probed with mouse antiserum or monoclonal antibodies. The detection of antigens reactive with the antibodies was performed by indirect enzyme-immunoassay using conjugated-anti-mouse immunoglobulins and a colour substrate. When antiserum raised to recombinant BVH-3 was tested against S. pneumoniae SP64 antigens, two reactive bands having apparent molecular masses of 127 kDa and 99 kDa were detected. Bands having the same apparent molecular masses were also detected when Mabs H3-1-F9, H3-1-D4, H3-1-H12, H11-1-E7, H11-1-H10 and H11-1.1-G11 were used individually as immunological probes. In contrast, Mabs specific for the BVH-3 molecule detected the 127 kDa band only and Mabs specific for BVH-11 detected the 99 kDa band only thus confirming the identity of the 127 and 99 kDa bands as BVH-3 and BVH-11, respectively. These studies provide evidence that BVH-3 and BVH-11 proteins are simultaneously present on S. pneumoniae. Moreover, the results are consistent with our previous observations that BVH-3 and BVH-11 possess epitopes that are common to both proteins and epitopes that are exclusive to either protein.

In S. pneumoniae SP64, mature BVH-3, BVH-11 and BVH-11-2 are proteins of 1019, 821 and 819 amino acids with predicted molecular mass of 112.5 kDa, 92.4 kDa, and 91.7 kDa, respectively. Although there is a discrepancy between the molecular mass predicted from the sequence and the molecular mass calculated on SDS-PAGE, BVH-3 can be distinguished from BVH-11 by its higher molecular mass. Moreover, BVH-3 molecules from S. pneumoniae strain SP63 have an apparent molecular mass of 112 kDa in SDS-PAGE compared to 127 kDa for BVH-3 of SP64 strain. This data is consistent with the deletion of a stretch of 177 amino acid residues in BVH-3 of S. pneumoniae strain SP63.

### EXAMPLE 11

This example describes the protection conferred in experimental infection of mice vaccinated with recombinant BVH-3 or BVH-11 gene products.

Groups of 7 or 8 female BALB/c mice (Charles River) were immunized subcutaneously three times at three-week intervals with either affinity purified thioredoxin-His•Tag-BVH-3M fusion protein, affinity purified thioredoxin-His•Tag-BVH-11M fusion protein or, as control, with Qui1A adjuvant alone in PBS. Twelve to 14 days following the third immunization, the mice were challenged intravenously with S. pneumoniae WU2 strain or intranasally with P4241 strain. Samples of the S. pneumoniae challenge inoculum were plated on chocolate agar plates to determine the CFU and to verify the challenge dose. The challenge dose was approximately 10⁶ CFU. Deaths were recorded for a period of 14 days and on day 14 post-challenge, the surviving mice were sacrificed and blood samples tested for the presence of S. pneumoniae organisms. The survival data are shown in Tables 6 and 7.

**Table 6. Protection mediated by recombinant BVH-3M and BVH-11M proteins in experimental infection with virulent S. pneumoniae WU2**

| Experiment | Immunogen | Alive : dead^{a} | Median days alive |
|---|---|---|---|
| 1 | BVH-3M | 8 : 0 | >14 |
| | none | 0 : 8 | 1 |
| 2 | BVH-11M | 8 : 0 | >14 |
| | none | 0 : 8 | 1 |

| | | | |
|---|---|---|---|
| ^{a} The number of mice alive : the number of mice dead on day 14 post-challenge. | | | |

**Table 7. Protection mediated by recombinant BVH-3M and BVH-11M proteins in experimental pneumonia with virulent S. pneumoniae P4241**

| Experiment | Immunogen | Alive : dead^{a} | Median day alive |
|---|---|---|---|
| 1 | BVH-3M | 6 : 1 | >14 |
| | none | 1 : 7 | 4.5 |
| 2 | BVH-3M | 8 : 0 | >14 |
| | BVH-11M | 8 : 0 | >14 |
| | none | 0 : 8 | 4 |

| | | | |
|---|---|---|---|
| ^{a} The number of mice alive : the number of mice dead on day 14 post-challenge. | | | |

All mice immunized with recombinant BVH-3M or BVH-11M protein survived to infection with WU2 while none of the control mice given adjuvant alone survived. All except one mice immunized with recombinant BVH-3M or BVH-11M protein survived to infection with P4241 while only one control mice given adjuvant alone survived. All hemocultures from surviving mice were negative at day 14 post-challenge. These results clearly indicate that both, BVH-3M and BVH-11M, elicit protective anti-pneumococcal immune responses in mice. The fact that these proteins are highly conserved among S. pneumoniae isolates emphasize the potential of BVH-3 and BVH-11 as universal vaccine candidates. Indeed, the BVH-3 and BVH-11 proteins from serogroup 6 S. pneumoniae strain SP64 elicited protection against pneumococcal infections with strains of different capsular serotypes.

Ideally, a vaccine that could protect against pneumococcal disease, could protect against meningitis, otitis media, bacteremia and pneumonia. BVH-3 and BVH-11 were protective against lethal systemic- and pneumonia-infection models thus suggesting that, in humans, BVH-3- and BVH11-protein-based vaccines could reduce the incidence of a wide spectrum of disease caused by virtually all S. pneumoniae independently of the capsular serotype.

Data from Tables 6 and 7 clearly demonstrate that BVH-3 and BVH-11 were, both, protection-eliciting molecules of S. pneumoniae. It was not known, however, whether protection can be mediated by specific sequences that were not shared on BVH-3 and BVH-11 molecules. Groups of female BALB/c mice (Charles River) were immunized subcutaneously three times at three-week intervals with either affinity purified thioredoxin-His•Tag- BVH-3AD, -BVH-3B or -BVH-3C fusion protein in presence of 15 µg of QuilA adjuvant (Cedarlane Laboratories Ltd, Hornby, Canada). Control mice were immunized with QuilA adjuvant alone in PBS or affinity purified thioredoxin-His•Tag or thioredoxin-His•Tag-fusion protein (His-Thio) in presence of Qui1A.

To determine the protective ability of a set of truncated proteins, termed NEW4, NEW5, NEW6, NEW7, NEW8, NEW9, NEW10, NEW11, NEW14 and BVH-11B, groups of female BALB/c mice (Charles River) were immunized subcutaneously two times at three-week intervals with 25 µg of either affinity purified His•Tag-fusion protein in presence of 15 µg of QuilA adjuvant. Ten to 14 days following the last immunization, the mice were challenged with virulent S. pneumoniae. Our results indicate that, BVH-3B, a truncated BVH-3 molecule consisting of amino acids 512-1039, elicited protection against the mouse-virulent strains WU2 and P4241. Similarly, BVH-11B, NEW4 and NEW5 molecules, three truncated BVH-11 molecules consisting of amino acids 354-840, amino acids 286-840 and amino acids 286-713, respectively, elicited protection against experiment intravenous challenge with WU2 and intranasal challenge with P4241. Moreover, vaccination with NEW10 and NEW14, consisting of amino acids 272-838 and amino acids 227-699 from BVH-11-2 molecule also resulted in protection against death with the pneumococcal strains. These results indicate that the region comprising 428 amino acids extending from amino acids 286-713 and amino acids 272-699 on S. pneumoniae SP64 BVH-11 and BVH-11-2 protein sequences, respectively, contains protective epitopes.
This region is highly conserved with a global 91% identity and 94% homology among thirteen BVH-11 protein sequences.

**Table 8. Evaluation of protection elicited by vaccination of mice with BVH-3 and BVH-11 gene products**

| | | Challenge with WU2 | | Challenge with P4241 | |
|---|---|---|---|---|---|
| Experiment | Immunogen | Alive : dead^{a} | Median day alive | Alive : dead | Median day alive |
| 1^{b} | None | 0 : 8 | 1 . 5 | 1 : 7 | 4 . 5 |
| | NEW4 | 8 : 0 | >14 | 8 : 0 | >14 |
| | NEW5 | 8 : 0 | >14 | 8 : 0 | >14 |
| | NEW7 | 0 : 8 | 2 | 0 : 8 | 5 |
| | BVH-11M | 8: 0 | >14 | 8 : 0 | >14 |
| 2^{b} | None | 0 : 8 | 1 | 0 : 8 | 4 |
| | NEW5 | 8 : 0 | >14 | 8 : 0 | >14 |
| | NEW8 | 0 : 8 | 1.5 | 0 : 8 | 5.5 |
| | NEW9 | 3 : 5 | 3.5 | 2 : 6 | 7 |
| | BVH-11M | 8 : 0 | >14 | 8 : 0 | >14 |
| 3^{b} | None | 0 : 8 | 1 | 0 : 8 | 4 |
| | NEW6 | 0: 8 | 1 | 4 : 4 | 10.5^{c} |
| | NEW10 | 8 : 0 | >14 | 8 : 0 | >14 |
| | NEW11 | 0 : 8 | 1.5 | 1 : 7 | 6 |
| | BVH-11M | 8 : 0 | >14 | 8 : 0 | >14 |
| 4^{b} | None | 0: 8 | 2 | 0 : 8 | 4 |
| | BVH-11B | 7 : 1 | >14 | 8 : 0 | >14 |
| | NEW14 | 8 : 0 | >14 | 8 : 0 | >14 |
| 5 | His-Thio | 0 : 8 | 2 | | |
| | BVH-3AD | 1 : 7 | 2.5 | | |
| | BVH-3B | 5 : 3 | >14 | | |
| 6 | His-Thio | 0 : 8 | 1 | | |
| | BVH-3C | 0: 8 | 1 | | |

| | | | | | |
|---|---|---|---|---|---|
| ^{a} The number of mice alive : the number of mice dead on day 14 post-challenge. ^{b} The WU2 challenge dose was 10⁵ CFU. ^{c} Mice living longer than 14 days were assigned a survival time of 14 days for the determination of median values. | | | | | |

### EXAMPLE 12

This example described the cloning and expression of a chimeric gene encoding for a chimeric polypeptide corresponding to the carboxy-terminal region of BVH-3 in fusion at the C' end to the carboxy-terminal region of BVH-11 and the additive protection observed after vaccination with a chimeric polypeptide.

It is clear from the studies described above that BVH-3 and BVH-11 are serologically distinct molecules simultaneously present on S. pneumoniae. The results of immunological studies of mice indicate that both proteins are good vaccine candidates. These proteins have the potential to provide protection against all pneumococci, regardless of serotype. Even though the two proteins share epitopes and sequences, they have different characteristics and may serve different biological functions. Thus, immunization against the two proteins may provide a higher level of protection than that imparted by each individually. To examine this, several avenues where full-length or truncated BVH-3 and BVH-11 are administered in combination or in conjugation can be explored. Here we describe the genetic engineering of a BVH-3-BVH-11 fusion gene and protein, termed NEW12 **(SEQ ID NO:76 and SEQ ID NO:58,** respectively), and the potential use of NEW12 protein as a vaccine.

BVH-3 and BVH-11 gene fragments corresponding to the 3'end of the genes were amplified by PCR using pairs of oligopucleotides engineered to amplify fragments spanning nucleotides 1414 to 3117 **(SEQ ID NO: 1)** and nucleotides 1060 to 2520 **(SEQ ID NO: 3)** from S. pneumoniae strain SP64 BVH-3 and BVH-11 genes, respectively. The primers used, HAMJ278 and HAMJ279; HAMJ282 and HAMJ283 had a restriction endonuclease site at the 5' end, thereby allowing directional in-frame cloning of the amplified product into the digested pET21b(+) plasmid vector (Table 2). PCR-amplified products were digested with restriction endonucleases and ligated to linearized plasmid pET21b(+) vector digested likewise. The resultant plasmid constructs were confirmed by nucleotide sequence analysis. The recombinant pET21b(+) plasmid containing the NdeI-HindIII BVH-3 PCR product was linearized by digestion with the restriction enzymes HindIII and NotI for the in-frame cloning of the HindIII-NotI DNA fragment obtained from the recombinant pET21(+) vector containing the BVH-11 gene fragment. Clones were first stabilized in E. coli DH5α before introduction into E. coli BL21(λDE3) for expression of a chimeric pneumococcal protein molecule. The recombinant chimeric polypeptide, termed NEW 12, was expressed as C-terminal fusion with an His-tag. The expressed recombinant NEW 12 protein was purified from supernatant fractions obtained from centrifugation of sonicated IPTG-induced E. coli cultures using a His-Bind metal chelation resin (QIAgen, Chatsworth, CA).

According to the same procedure described above, it is possible to construct other chimeric polypeptides, as a result of a simultaneous expression of New 1 and New 4, New 1 and New 5, New 1 and New 10, or New 1 and New 14. The construction can be with New 1 upstream or downstream of New 4, New 5, New 10, BVH-11B or New 14. It is also possible to construct other chimeric polypeptides as a result of a simultaneous expression of more than two fragments of either genes of BVH-3, BVH-11 or BVH-11-2.

Groups of 8 female BALB/c mice (Charles River) were immunized subcutaneously two times at three-week intervals with 25 µg of either affinity purified His•Tag-fusion NEW1, BVH-11B or NEW12 protein in presence of 15 µg of QuilA adjuvant. Ten to 14 days following the last immunization, the mice were challenged with virulent S. pneumoniae. As demonstrated before, NEW1 and BVH-11B molecules comprising amino acids 472 to 1039 from BVH-3 protein and amino acids 354-840 from BVH-11 protein, respectively, correspond to portions of the proteins capable of eliciting a protective immune response. To determine if a chimeric polypeptide would significantly improve the protection compared with those seen for the individual counterparts, the challenge dose was adjusted in a manner that protection was not expected with NEW1 and BVH-11B molecules. Interestingly, the chimeric NEW12 protein, elicited protection against the mouse-virulent strains WU2 and P4241. Seven out of 8 mice immunized with NEW12 were still alive 10 days after the challenge while 28 out of 32 mice immunized with NEW1, BVH-11B, BVH-3M or adjuvant alone were dead by five days post-challenge. Thus, vaccination of mice with NEW12 provided the highest degree of protection against WU2 challenge. These results indicate that immunization with a chimeric polypeptide and possibly a combination of BVH-3 and BVH-11 gene products can provide additional protection to that obtained by administration of BVH-3 or BVH-11 antigens alone.

**Table 9. Evaluation of protection elicited by vaccination of mice with the chimeric NEW12 molecule**

| | Challenge with WU2 | | Challenge with P4241 | |
|---|---|---|---|---|
| Immunogen | Alive : dead^{a} | Median day alive | Alive : dead | Median day alive |
| None | 0 : 8 | 1 | 0 : 8 | 5 |
| NEW1' | 2 : 6 | 2 | 1 : 7 | 8 |
| BVH-11B | 1 : 7 | 3.5 | 8 : 0 | >14 |
| NEW12 | 6 : 2 | >14 | 7 : 1 | >14 |
| BVH-3M | 1: 7 | 3 | 8 : 1 | >14 |

### EXAMPLE 13

This example illustrates the identification of additional BVH-3 and BVH-11 related sequences in Streptococcus species other than S. pneumoniae.

It was previously shown that BVH-3, BVH-11 and BVH-11-2 are a family of related proteins sharing common sequences. Homology searches were performed with the nucleotide sequence from the conserved region of these genes and compared with GenBank and EMBL sequences using FASTA. The most significant homology was observed with a 2.469-kb gene coding for a calculated 92-kDa protein (**SEQ ID NO: 81**) of unknown function in S. agalactiae also called group B streptococcus or GBS. The gene was designated BVH-71. A protein demonstrating 99.2% identity and 99.5% similarity with that of GBS was also identified in S. pyogenes also called group A streptococcus or GAS **(SEQ ID NO: 83).** The 5' region of the BVH-71 sequences **(SEQ ID NO: 80** and **SEQ ID NO: 82**), spanning nucleotides 1 to 717, demonstrated 58 and 60% identity with the conserved regions of BVH-3 (nucleotides 1 to 675) and BVH-11 (nucleotides 1 to 684) genes respectively. The first 239 amino acids of the translated sequences of the GBS and GAS BVH-71 open reading frames are 51 and 54% identical to the first 225 and 228 amino acids of BVH-3 and BVH-11, respectively. In addition to structural similarities, streptococcal BVH-3, BVH-11 and BVH-71 proteins also share antigenic epitopes. A 97-kDa band was revealed on Western blots of GAS or GBS whole cells, using Mab H11-1.1-G11 reactive with the BVH-3 and BVH-11 conserved regions. Similarly, GAS and GBS recombinant BVH-71 proteins were detected in Western immunoblot analysis.
These results indicate that BVH-71, BVH-3 and BVH-11 proteins might share similar functions. Our results also suggest that BVH-71 proteins can be used as protein vaccine components of anti-streptococcus. In a further embodiment BVH-71 proteins can be used as protein vaccine components of anti-GAS or anti-GBS vaccines.

## Claims

1. An isolated polynucleotide encoding a polypeptide having at least 95% identity to any one of SEQ ID NOs: 2, 8, 10, 16, 55, 64-66 or 78 wherein the polypeptide elicits an anti-streptococcal immune response when administered to an individual.

2. An isolated polynucleotide encoding a polypeptide capable of inducing the generation of antibodies having binding specificity for a polypeptide having an amino acid sequence of any one of SEQ ID NOs: 2, 8, 10, 16, 55, 64-66 or 78.

3. An isolated polynucleotide that is complementary to the polynucleotide of claim 1 or 2.

4. The polynucleotide of any one of claims 1 to 3, wherein said polynucleotide is DNA.

5. The polynucleotide of any of claims 1 or 3 wherein said polynucleotide is RNA.

6. A vector comprising the polynucleotide of any of claims 1 to 5, wherein said polysaccharide is operably linked to an expression control region.

7. A host cell transformed with a vector of claim 6.

8. A process for producing a polypeptide comprising culturing a host cell according to claim 7 under conditions suitable for expression of said polypeptide.

9. An isolated polypeptide which elicits an anti-streptococcal immune response when administered to an individual, said polypeptide having at least 95% identity to any one of SEQ ID NOs: 2, 8, 10, 16, 55, 64-66 or 78.

10. An isolated polypeptide which elicits an anti-streptococcal immune response when administered to an individual, and which is capable of inducing the generation of antibodies having binding specificity for a polypeptide having an amino acid sequence of any one of SEQ ID NOs: 2, 8, 10, 16, 55, 64-66 or 78.

11. An isolated polypeptide having an amino acid sequence selected from SEQ ID NOs: 2, 8, 10, 16, 55, 64-66 or 78.

12. An isolated polypeptide according to claim 11, wherein the N-terminal Met is deleted.

13. An isolated polypeptide according to claim 11, wherein the secretory amino acid sequence is deleted.

14. A chimeric polypeptide which elicits an anti-streptococcal immune response when administered to an individual comprising two or more polypeptides selected from SEQ ID NOs: 2, 8,10, 16, 55, 64-66 or 78 wherein the two or more polypeptides are linked to form a chimeric polypeptide.

15. A chimeric polypeptide as claimed in claim 14 comprising polypeptides having the amino acid sequence of SEQ ID NOs: 10 and 64 wherein the polypeptides are linked to form a chimeric polypeptide.

16. A chimeric polypeptide of formula (I) which elicits an anti-streptococcal immune response when administered to an individual:
A-(B)m-(C)n-D (I)
Wherein
M is 0 or 1,
N is 0 or 1,
A is selected from SEQ ID NOs: 2, 8, 10, 16, 55, 64-66 or 78;
B is chosen from SEQ ID NOs: 2, 8, 10, 16, 55, 64-66 or 78;
C is chosen from SEQ ID NOs: 2, 8, 10, 16, 55, 64-66 or 78; and
D is chosen from SEQ ID NOs: 2, 8, 10, 16, 55, 64-66 or 78.

17. A chimeric polypeptide according to claim 16, wherein the chimeric peptide has the formula (I):
A-(B)m-(C)n-D (I)
Wherein
m is 0 or 1,
n is 0 or 1, and
each of A, B, C and D are independently of one another SEQ ID NO: 10 or 64.

18. A vaccine composition comprising a polypeptide according to any one of claims 9 to 17 and a pharmaceutically acceptable carrier, diluent or adjuvant.

19. Use of a vaccine composition according to clam 18 for the manufacture of a medicament for the therapeutic or prophylactic treatment of meningitis, otitis media, bacteremia or pneumonie infection in an individual susceptible to meningitis, otitis media, bacteremia or pneumonia infection wherein a therapeutic or prophylactic amount of said medicament is to be administered to said individual.

20. Use according to claim 19, wherein said individual is a mammal.

21. Use according to claim 19 or 20 , wherein said bacterial infection is S. pneumoiae, group A streptococcus (Pyogenes), group B streptococcus (GBS or agalactiae), dysgalactiae, uberis, nocardia or Staphylococcus aureus.

22. Use according to claim 21, wherein said bacterial infection is S. pneumoniae.

23. Use of the vaccine composition according to claim 18 for the manufacture of a medicament fo the prophylactic or therapeutic treatment of streptococcal infection in an animla susceptible to or infected with streptococcal infection comprising administering to said animal a prophylactic or therapeutic amount of the composition.

24. Use according to claim 23, wherein said individual is a human.

## Patentansprüche

1. Isoliertes Polynukleotid, codierend ein Polypeptid mit einer mindestens 95 %igen Identität zu einer der SEQ ID Nrn. 2, 8, 10, 16, 55, 64-66 oder 78, wobei das Polypeptid eine Antistreptokokken-Immunreaktion hervorruft, wenn es einem Individuum verabreicht wird.

2. Isoliertes Polynukleotid, codierend ein Polypeptid, das eine Erzeugung von Antikörpern mit einer Bindungsspezifizität für ein Polypeptid mit einer Aminosäuresequenz von einer der SEQ ID Nrn. 2, 8, 10, 16, 55, 64-66 oder 78 induzieren kann.

3. Isoliertes Polynukleotid, das zu dem Polynukleotid von Anspruch 1 oder 2 komplementär ist.

4. Polynukleotid gemäß einem der Ansprüche 1 bis 3, wobei das Polynukleotid DNA ist.

5. Polynukleotid gemäß einem der Ansprüche 1 bis 3, wobei das Polynukleotid RNA ist.

6. Vektor, umfassend das Polynukleotid gemäß einem der Ansprüche 1 bis 5, wobei das Polysaccharid operabel an eine Expressionskontrollregion gebunden ist.

7. Wirtszelle, transformiert mit einem Vektor gemäß Anspruch 6.

8. Verfahren zur Erzeugung eines Polypeptids, umfassend die Kultivierung einer Wirtszelle gemäß Anspruch 7 unter geeigneten Bedingungen zur Expression des Polypeptids.

9. Isoliertes Polypeptid, das eine Antistreptokokken-Immunreaktion hervorruft, wenn es einem Individuum verabreicht wird, wobei das Polypeptid eine mindestens 95 %ige Identität zu einer der SEQ ID Nrn. 2, 8, 10, 16, 55, 64-66 oder 78 aufweist.

10. Isoliertes Polypeptid, das eine Antistreptokokken-Immunreaktion hervorruft, wenn es einem Individuum verabreicht wird und das dazu in der Lage ist, die Erzeugung von Antikörpern zu induzieren, die eine Bindungsspezifizität für ein Polypeptid mit einer Aminosäuresequenz von einer der SEQ ID Nrn. 2, 8, 10, 16, 55, 64-66 oder 78 aufweisen.

11. Isoliertes Polypeptid mit einer Aminosäuresequenz ausgewählt aus den SEQ ID Nrn. 2, 8, 10, 16, 55, 64-66 oder 78.

12. Isoliertes Polypeptid gemäß Anspruch 11, wobei das N-terminale Met deletiert ist.

13. Isoliertes Polypeptid gemäß Anspruch 11, wobei die sekretorische Aminosäuresequenz deletiert ist.

14. Chimäres Polypeptid, das eine Antistreptokokken-Immunreaktion hervorruft, wenn es einem Individuum verabreicht wird, umfassend zwei oder mehr Polypeptide ausgewählt aus den SEQ ID Nrn. 2, 8, 10, 16, 55, 64-66 oder 78, wobei die zwei oder mehr Polypeptide zur Bildung eines chimären Polypeptids verbunden sind.

15. Chimäres Polypeptid gemäß Anspruch 14, umfassend Polypeptide mit der Aminosäuresequenz der SEQ ID Nrn. 10 und 64, wobei die Polypeptide zur Bildung eines chimären Polypeptids verbunden sind.

16. Chimäres Polypeptid gemäß Formel (I), das eine Antistreptokokken-Immunreaktion hervorruft, wenn es einem Individuum verabreicht wird:
A-(B)m-(C)n-D (I)
worin
m 0 oder 1 ist,
n 0 oder 1 ist,
A ist ausgewählt aus SEQ ID Nrn. 2, 8, 10, 16, 55, 64-66 oder 78,
B ist ausgewählt aus SEQ ID Nrn. 2, 8, 10, 16, 55, 64-66 oder 78,
C ist ausgewählt aus SEQ ID Nrn. 2, 8, 10, 16, 55, 64-66 oder 78 und
D ist ausgewählt aus SEQ ID Nrn. 2, 8, 10, 16, 55, 64-66 oder 78.

17. Chimäres Polypeptid gemäß Anspruch 16, wobei das chimäre Peptid die Formel (I) aufweist:
A-(B)m-(C)n-D (I)
worin
m 0 oder 1 ist,
n 0 oder 1 ist und
jedes von A, B, C und D ist unabhängig voneinander SEQ ID Nr. 10 oder 64.

18. Vakzinzusammensetzung, umfassend ein Polypeptid gemäß einem der Ansprüche 9 bis 17 und einen pharmazeutisch annehmbaren Träger, ein Verdünnungsmittel oder ein Adjuvans.

19. Verwendung einer Vakzinzusammensetzung gemäß Anspruch 18 für die Herstellung eines Medikaments für die therapeutische oder prophylaktische Behandlung der Meningitis, Otitis media, Bakteriämie oder Pneumonieinfektion bei einem Individuum, das gegenüber einer Meningitis, Otitis media, Bakteriämie oder Pneumonieinfektion empfänglich ist, wobei eine therapeutische oder prophylaktische Menge des Medikaments dem Individuum zu verabreichen ist.

20. Verwendung gemäß Anspruch 19, wobei das Individuum ein Säuger ist.

21. Verwendung gemäß Anspruch 19 oder 20, wobei die bakterielle Infektion von S. pneumoniae, Gruppe A Streptococcus (pyogenes), Gruppe B Streptococcus (GBS oder agalactiae), dysgalactiae, uberis, Nocardia oder Staphylococcus aureus ausgelöst wird.

22. Verwendung gemäß Anspruch 21, wobei die bakterielle Infektion von S. pneumoniae ausgelöst wird.

23. Verwendung der Vakzinzusammensetzung gemäß Anspruch 18 für die Herstellung eines Medikaments für die prophylaktische oder therapeutische Behandlung einer Streptokokkeninfektion bei einem Tier, das gegenüber einer Streptokokkeninfektion empfänglich oder **dadurch** infiziert ist, umfassend die Verabreichung einer prophylaktischen oder therapeutischen Menge der Zusammensetzung an das Tier.

24. Verwendung gemäß Anspruch 23, wobei das Individuum ein Mensch ist.

## Revendications

1. Polynucléotide isolé codant pour un polypeptide ayant une identité d'au moins 95 % à l'une quelconque des SEQ ID No.: 2, 8, 10, 16, 55, 64-66 ou 78, dans lequel le polypeptide déclenche une réponse immunitaire antistreptococcique quand il est administré à un individu.

2. Polynucléotide isolé codant pour un polypeptide capable d'induire la génération d'anticorps ayant une spécificité de liaison pour un polypeptide ayant la séquence d'acides aminés de l'une quelconque des SEQ ID No.: 2, 8, 10, 16, 55, 64-66 ou 78.

3. Polynucléotide isolé qui est complémentaire du polynucléotide selon la revendication 1 ou 2.

4. Polynucléotide selon l'une quelconque des revendications 1 à 3, dans lequel ledit polynucléotide est l'ADN.

5. Polynucléotide selon l'une quelconque des revendications 1 à 3, dans lequel ledit polynucléotide est l'ARN.

6. Vecteur comprenant le polynucléotide selon l'une quelconque des revendications 1 à 5, dans lequel ledit polynucléotide est lié de manière opérationnelle à une région de contrôle de l'expression.

7. Cellule hôte transformée avec un vecteur selon la revendication 6.

8. Procédé de production d'un polypeptide comprenant la culture d'une cellule hôte selon la revendication 7 dans des conditions favorables à l'expression dudit polypeptide.

9. Polypeptide isolé qui déclenche une réponse immunitaire antistreptococcique quand il est administré à un individu, ledit polypeptide ayant une identité d'au moins 95 % à l'une quelconque des SEQ ID No.: 2, 8, 10, 16, 55, 64-66 ou 78.

10. Polypeptide isolé qui déclenche une réponse immunitaire antistreptococcique quand il est administré à un individu, et qui est capable d'induire la génération d'anticorps ayant une spécificité de liaison pour un polypeptide ayant la séquence d'acides aminés de l'une quelconque des SEQ ID No.: 2, 8, 10, 16, 55, 64-66 ou 78.

11. Polypeptide isolé ayant une séquence d'acides aminés choisie parmi SEQ ID No.: 2, 8, 10, 16, 55, 64-66 ou 78.

12. Polypeptide isolé selon la revendication 11, dans lequel le résidu Met N-terminal est délété.

13. Polypeptide isolé selon la revendication 11, dans lequel la séquence d'acides aminés sécrétoire est délétée.

14. Polypeptide chimérique qui déclenche une réponse immunitaire antistreptococcique quand il est administré à un individu comprenant deux polypeptides ou plus choisis parmi SEQ ID No.: 2, 8, 10, 16, 55, 64-66 ou 78, dans lequel les deux polypeptides ou plus sont liés pour former un polypeptide chimérique.

15. Polypeptide chimérique selon la revendication 14 comprenant des polypeptides ayant la séquence d'acides aminés de SEQ ID No.: 10 et 64, dans lequel les polypeptides sont liés pour former un polypeptide chimérique.

16. Polypeptide chimérique de formule (I) qui déclenche une réponse immunitaire antistreptococcique quand il est administré à un individu :
A-(B)m-(C)n-D (I)
dans laquelle
m est 0 ou 1,
n est 0 ou 1,
A est choisi parmi SEQ ID No.: 2, 8, 10, 16, 55, 64-66 ou 78 ;
B est choisi parmi SEQ ID No.: 2, 8, 10, 16, 55, 64-66 ou 78 ;
C est choisi parmi SEQ ID No.: 2, 8, 10, 16, 55, 64-66 ou 78 ; et
D est choisi parmi SEQ ID No.: 2, 8, 10, 16, 55, 64-66 ou 78.

17. Polypeptide chimérique selon la revendication 16, dans lequel le peptide chimérique répond à la formule (I) :
A-(B)m-(C)n-D (I)
dans laquelle
m est 0 ou 1,
n est 0 ou 1, et
chacun des A, B, C et D est indépendamment l'un de l'autre SEQ ID No.: 10 ou 64.

18. Composition vaccinale comprenant un polypeptide selon l'une quelconque des revendications 9 à 17 et un véhicule, un diluant ou un adjuvant pharmaceutiquement acceptable.

19. Utilisation d'une composition vaccinale selon la revendication 18 pour la fabrication d'un médicament pour le traitement thérapeutique ou prophylactique de la méningite, de l'otite moyenne, d'une bactériémie ou d'une infection à pneumoniae chez un individu sensible à la méningite, à l'otite moyenne, à la bactériémie ou à l'infection à pneumoniae, dans laquelle une quantité thérapeutique ou prophylactique dudit médicament doit être administrée audit individu.

20. Utilisation selon la revendication 19, dans laquelle ledit individu est un mammifère.

21. Utilisation selon la revendication 19 ou 20, dans laquelle ladite infection bactérienne est à S. pneumoniae, streptococcus du groupe A (Pyogenes), streptococcus du groupe B (GBS ou agalactiae), dysgalactiae, uberis, nocardia ou Staphylococcus aureus.

22. Utilisation selon la revendication 21, dans laquelle ladite infection bactérienne est à S. pneumoniae.

23. Utilisation de la composition vaccinale selon la revendication 18 pour la fabrication d'un médicament pour le traitement prophylactique ou thérapeutique d'une infection à streptocoques chez un animal sensible à, ou souffrant d'une infection à streptocoques comprenant l'administration audit animal d'une quantité prophylactique ou thérapeutique de la composition.

24. Utilisation selon la revendication 23, dans laquelle ledit individu est l'homme.
